# EUROPEAN PATENT SPECIFICATION

(11) **EP 2 852 572 B1**
(45) Date of publication and mention of the grant of the patent: **01.04.2020**
(21) Application number: 12783780.5
(22) Date of filing: 25.10.2012
(51) Int. Cl.: C07D 209/80, C07D 403/12, C07B 59/00, C07D 471/04, C07D 471/14, C07D 209/88

(54) **CARBOLINE AND CARBAZOLE BASED IMAGING AGENTS FOR DETECTING NEUROLOGICAL DYSFUNCTION**
AUF CARBOLIN UND CARBAZOL BASIERENDE BILDGEBUNGSMITTEL FÜR DEN NACHWEIS NEUROLOGISCHER DYSFUNKTIONEN
AGENTS D'IMAGERIE À BASE DE CARBOLINE ET DE CARBAZOLE POUR LA DÉTECTION DE DYSFONCTION NEUROLOGIQUE

(30) Priority: 22.05.2012 US 201213477095
(43) Date of publication of application: 01.04.2015
(73) Proprietor: Eli Lilly and Company, Indianapolis, IN 46285 (US)
(72) Inventor: SZARDENINGS, Anna Katrin, Torrance, California 90503 (US); KOLB, Hartmuth C., Playa Del Rey, California 90293 (US); WALSH, Joseph C., Pacific Palisades, California 90272 (US); CHEN, Gang, Rancho Paios Verdes, California 90275 (US); GANGADHARMATH, Umesh B., Los Angeles, California 90034 (US); KASI, Dhanalakshmi, Silver Spring, MD 20906-6012 (US); LIU, Changhui, Los Angeles, California 90036 (US); SINHA, Anjana, San Diego, California 92130 (US); WANG, Eric, San Diego, California 92130 (US); YU, Chul, Los Angeles, California 90005 (US); ZHANG, Wei, Los Angeles, California 90036 (US); CHEN, Kai, San Gabriel, California 91776 (US); MOCHARLA, Vani P., Los Angeles, California 90034 (US); SCOTT, Peter J.H., Ypsilanti, Michigan 48197 (US)
(74) Representative: O'Connor, David
(86) International application number: PCT/US2012/061912
(87) International publication number: WO 2013/176698

(56) References cited:
- EP-A2- 2 511 006
- WO-A1-2006/125887
- WO-A1-2008/132454
- WO-A1-2009/102498
- WO-A1-2011/119565
- WO-A2-2006/005063
- WO-A2-2010/011964
- WO-A2-2010/111303
- DE-A1-102006 062 203
- JP-A- 2012 089 777
- M. NAYA ET AL: "Myocardial -Adrenergic Receptor Density Assessed by 11C-CGP12177 PET Predicts Improvement of Cardiac Function After Carvedilol Treatment in Patients with Idiopathic Dilated Cardiomyopathy", THE JOURNAL OF NUCLEAR MEDICINE, vol. 50, no. 2, 1 February 2009 (2009-02-01), pages 220-225, XP055045770, ISSN: 0161-5505, DOI: 10.2967/jnumed.108.056341
- P. DOZE ET AL: "Synthesis and evaluation of (S)-[18F]-fluoroethylcarazolol for in vivo [beta]-adrenoceptor imaging in the brain", NEUROCHEMISTRY INTERNATIONAL, vol. 41, no. 1, 1 July 2002 (2002-07-01), pages 17-27, XP055045765, ISSN: 0197-0186, DOI: 10.1016/S0197-0186(01)00140-1
- JOOST BART ET AL: "New positron emission tomography tracer [ 11 C]carvedilol reveals P-glycoprotein modulation kinetics", BRITISH JOURNAL OF PHARMACOLOGY, vol. 145, no. 8, 1 August 2005 (2005-08-01), pages 1045-1051, XP055045763, ISSN: 0007-1188, DOI: 10.1038/sj.bjp.0706283
- ELDER S T ET AL: "Esters of 6-(4'-fluorobenzylamino)-.beta.-carboline- 3-carboxylic acid as potential benzodiazepine imaging agents for PET", JOURNAL OF LABELLED COMPOUNDS AND RADIOPHARMACEUTICALS, JOHN WILEY, CHICHESTER, GB, vol. 36, no. 3, 1 January 1995 (1995-01-01), pages 205-211, XP002487251, ISSN: 0362-4803, DOI: 10.1002/JLCR.2580360302
- DATABASE CAPLUS [Online] CHEMICAL ABSTRACTS SERVICE, COLUMBUS, OHIO, US; 16 November 1984 (1984-11-16), "9-Azafluorene", XP002688209, retrieved from STN Database accession no. 86-74-8
- "2-FLUORO-7-IODO-9H-CARBAZOLE", CROSSFIRE BEILSTEIN, 1994, XP002234312,
- "2-METHOXY-7-METHYL-9H-CARBAZOLE", CROSSFIRE BEILSTEIN, 1984, XP002234313,
- NAKAMURA ET AL: "O incorporation from H2<18>O2 in the oxidation of N-methylcarbazole and sulfides catalyzed by microperoxidase-11", TETRAHEDRON LETTERS, ELSEVIER, AMSTERDAM, NL, vol. 33, no. 37, 8 September 1992 (1992-09-08), pages 5409-5412, XP022084202, ISSN: 0040-4039, DOI: 10.1016/S0040-4039(00)79107-6
- DATABASE REGISTRY [Online] CHEMICAL ABSTRACTS SERVICE, COLUMBUS, OHIO, US; 18 August 1998 (1998-08-18), "1-methyl- (9CI)5H-Pyrido[4,3-b]indol-3-amine-3-14C", XP002695118, retrieved from STN Database accession no. 210049-14-2
- DATABASE REGISTRY [Online] CHEMICAL ABSTRACTS SERVICE, COLUMBUS, OHIO, US; 18 August 1998 (1998-08-18), "1,4-dimethyl-5H-Pyrido[4,3-b]indol-3-amin e-3-14C", XP002695119, retrieved from STN Database accession no. 210049-07-3
- DATABASE REGISTRY [Online] CHEMICAL ABSTRACTS SERVICE, COLUMBUS, OHIO, US; "1,4-dimethyl-5H-Pyrido[4,3-b]indol-3-amin e-3-14C monoacetate", XP002695120, retrieved from STN Database accession no. 210049-08-4
- DATABASE REGISTRY [Online] CHEMICAL ABSTRACTS SERVICE, COLUMBUS, OHIO, US; "1-methyl- (9CI)5H-Pyrido[4,3-b]indol-3-amine-3-14C monoacetate", XP002695121, Database accession no. 210049-15-3
- SHIGEO MANABE ET AL: "Carcinogenic tryptophan pyrolysis products in human lens", EXPERIMENTAL EYE RESEARCH, vol. 48, no. 3, 1 March 1989 (1989-03-01), pages 351-363, XP055058834, ISSN: 0014-4835, DOI: 10.1016/S0014-4835(89)80004-1
- JING CHEN ET AL: "Microwave-Enhanced Fischer Reaction: An Efficient One-Pot Synthesis of [gamma]-Carbolines", SYNLETT, vol. 2008, no. 1, 1 January 2008 (2008-01-01), pages 77-82, XP055058782, ISSN: 0936-5214, DOI: 10.1055/s-2007-992411
- DATABASE CA [Online] CHEMICAL ABSTRACTS SERVICE, COLUMBUS, OHIO, US; 2000, STOLLE, W. T. ET AL: "The preparation of isotopically labeled 2,4,6-trisubstituted pyrimidines", XP002695122, retrieved from STN Database accession no. 2002:174788
- DATABASE CA [Online] CHEMICAL ABSTRACTS SERVICE, COLUMBUS, OHIO, US; 2001, EASTER, JOHN A.: "Synthesis of isotopically labeled 1-[(2,4-di-1-pyrrolidinyl-9H- pyrimido[4,5-b]indol-9-yl)acetyl]pyrrolidi ne monohydrochloride, PNU-142731a, an orally active antiasthma agent", XP002695123, retrieved from STN Database accession no. 2000:902443
- DATABASE CA [Online] CHEMICAL ABSTRACTS SERVICE, COLUMBUS, OHIO, US; 10 September 1999 (1999-09-10), EASTER, JOHN A. ET AL: "Synthesis of several isotopically labeled pyrrolo[1,3-d]pyrimidine analogs", XP002695124, retrieved from STN Database accession no. 2001:743994
- JOHN A. HYATT ET AL: "Facile synthesis of 9H-pyrimido[4,5-b]indoles from photolysis of 8-phenyltetrazolo[1,5-c]pyrimidines in acidic media", THE JOURNAL OF ORGANIC CHEMISTRY, vol. 37, no. 21, 1 October 1972 (1972-10-01), pages 3216-3220, XP055058974, ISSN: 0022-3263, DOI: 10.1021/jo00986a005
- BLOM ELISABETH ET AL: "Synthesis and in vitro evaluation of 18F-.beta.-carboline alkaloids as PET ligands", JOURNAL OF LABELLED COMPOUNDS AND RADIOPHARMACEUTICALS, JOHN WILEY, CHICHESTER, GB, vol. 51, no. 6, 1 May 2008 (2008-05-01), pages 277-282, XP002528623, ISSN: 0362-4803, DOI: 10.1002/JLCR.1519

## Description

### BACKGROUND

Alzheimer's disease (AD), a leading cause of dementia, develops in one percent of the population between the ages 65 and 69, and increasing to 40-50% in those 95 years and older. AD patients exhibit telltale clinical symptoms that include cognitive impairment and deficits in memory function. In these patients, heavy senile plaque burden found in the cerebral cortex, verified by post mortem histopathological examination, confirms the presence of AD. The mature senile plaques consist of intracellular neurofibrillary tangles (NFT) derived from filaments of hyperphosphorylated tau proteins, and extracellular β-amyloid peptides derived from enzymatic processing of amyloid precursor protein. Interestingly, despite the development and presence of senile plaques in elderly persons with normal cognitive function, the severity of NFT and senile plaque deposition purportedly correlates with a loss of cognitive function and neuronal circuitry deterioration.

Neurological imaging of AD has seen the emergence of imaging tracers that appear to confirm the presence of AD based on plaque and fibril mediated tracer uptake and, subsequently, are currently undergoing extensive clinical examination. Many of these tracers contain chemotypes that derive from fluorescent dyes (Table 1).

The current array of AD imaging agents can only confirm the well-established manifestation of AD and this late stage diagnosis offers little defense against further disease progression past 36 months. Secondly, the detection of senile plaques and tangles may not correlate to development of the early stages of AD. Recent data suggests that the amyloid cascade model [Hardy, J. and D. Selkoe, The Amyloid Hypothesis of Alzheimer's Disease: Progress and Problems on the Road to Therapeutics. Science, 2002. 297: p. 353-356] does not accurately depict the primary factors leading to cognitive decline in AD patients and that other contributing factors, such as neuorotoxic soluble oligomers and aggregates may play a contributory role in neurodegeneration. [Talaga, P., Inhibitors of beta-amyloid aggregation: still an issue of structure and function? Drug Discovery Today: Therapeutic Strategies, 2004. 1: p. 7-12]. To date, FDDNP and PIB are not known to bind to neurotoxic soluble oligomers and aggregates and thus are not expected to differentiate accurately between the early stages of AD from the advanced stages of AD in patients.

A number of medical diagnostic procedures, including PET and SPECT utilize radiolabeled compounds. PET and SPECT are very sensitive techniques and require small quantities of radiolabeled compounds, called tracers. The labeled compounds are transported, accumulated and converted *in vivo* in exactly the same way as the corresponding non-radioactively compound. Tracers, or probes, can be radiolabeled with a radionuclide useful for PET imaging, such as ¹¹C, ¹³N, ¹⁵O, ¹⁸F, ⁶⁴Cu and ¹²⁴I, or with a radionuclide useful for SPECT imaging, such as ⁹⁹Tc, ⁷⁷Br, ⁶¹Cu, ¹⁵³Gd, ¹²³I, ¹²⁵I, ¹³¹I and ³²P.

PET creates images based on the distribution of molecular imaging tracers carrying positron-emitting isotopes in the tissue of the patient. The PET method has the potential to detect malfunction on a cellular level in the investigated tissues or organs. PET has been used in clinical oncology, such as for the imaging of tumors and metastases, and has been used for diagnosis of certain brain diseases, as well as mapping brain and heart function. Similarly, SPECT can be used to complement any gamma imaging study, where a true 3D representation can be helpful, for example, imaging tumor, infection (leukocyte), thyroid or bones.

WO 2009/102498 discloses radiolabeled compounds and methods of diagnosing Alzheimer's disease.

WO 2011/119565 and WO 2010/111303 disclose imaging agents and methods for detecting neurological disorders.

Database Registry, Chemical Abstracts Service, Columbus, Ohio, US; 18 August 1998, 210049-14-2 discloses 1-methyl-5H-Pyrido[4,3-b]indol-3-amine-3-¹⁴C.

Database Registry, Chemical Abstracts Service, Columbus, Ohio, US; 18 August 1998, 210049-07-3 discloses 1,4-dimethyl-5H-Pyrido[4,3-b]indol-3-amine-3-¹⁴C.

Database Registry, Chemical Abstracts Service, Columbus, Ohio, US; 18 August 1998, 210049-08-4 discloses 1,4-dimethyl-5H-Pyrido[4,3-b]indol-3-amine-3-¹⁴C monoacetate.

Database Registry, Chemical Abstracts Service, Columbus, Ohio, US; 18 August 1998, 210049-15-3 discloses 1-methyl-5H-Pyrido[4,3-b]indol-3-amine-3-¹⁴C monoacetate. Manabe, S. et al., Exp. Eye Res., 48(3), 1989, 351-363 discloses 3-amino-1,4-dimethyl-5H-pyrido-[4,3-b]indole and 3-amino-1-methyl-5H-pyrido[4,3-b]indole as carcinogenic tryptophan pyrolysis products in human lens.

JP 2012 089777 discloses organic electroluminescence elements.

Chen, J. et al., Synlett, 2008, 77-82 discloses a one-pot synthesis of γ-carbolines.

WO 2006/125887 discloses 5H-pyrido[4,3-b]indole derivative compounds for treating polyglutamin neurodegenerative diseases.

### BRIEF DESCRIPTION OF DRAWINGS

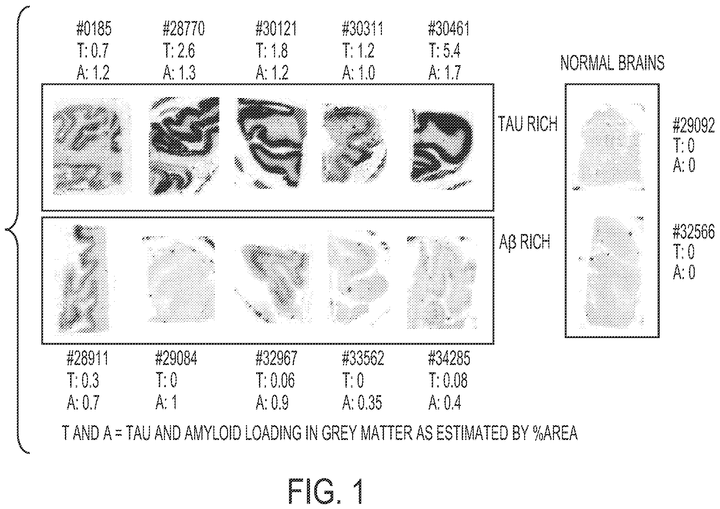

Figure 1 shows Audoradiography of [18F]-T794.

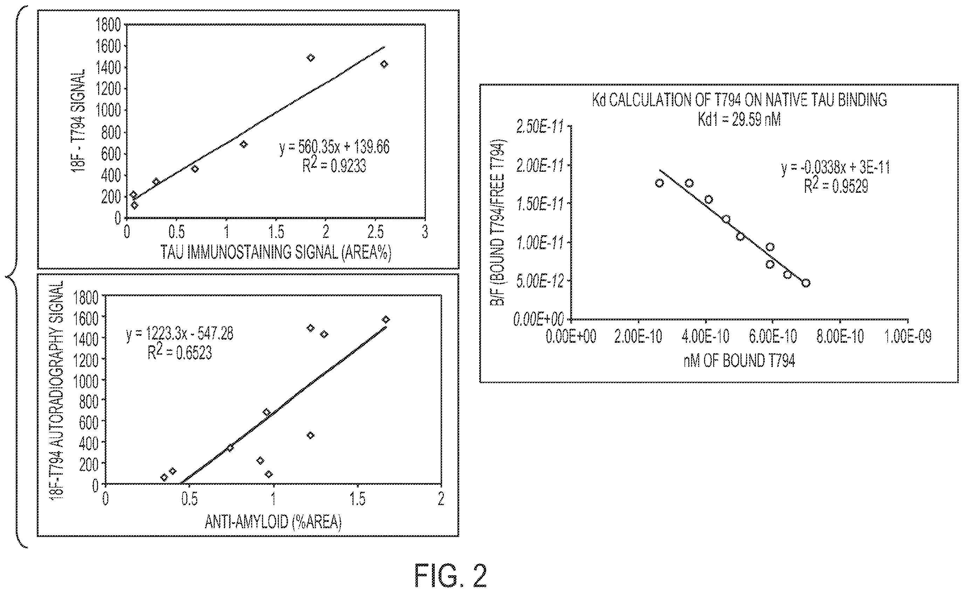

Figure 2 shows the correlation of [18F]-T794 with Tau and Amyloid loads and KD (30nM).

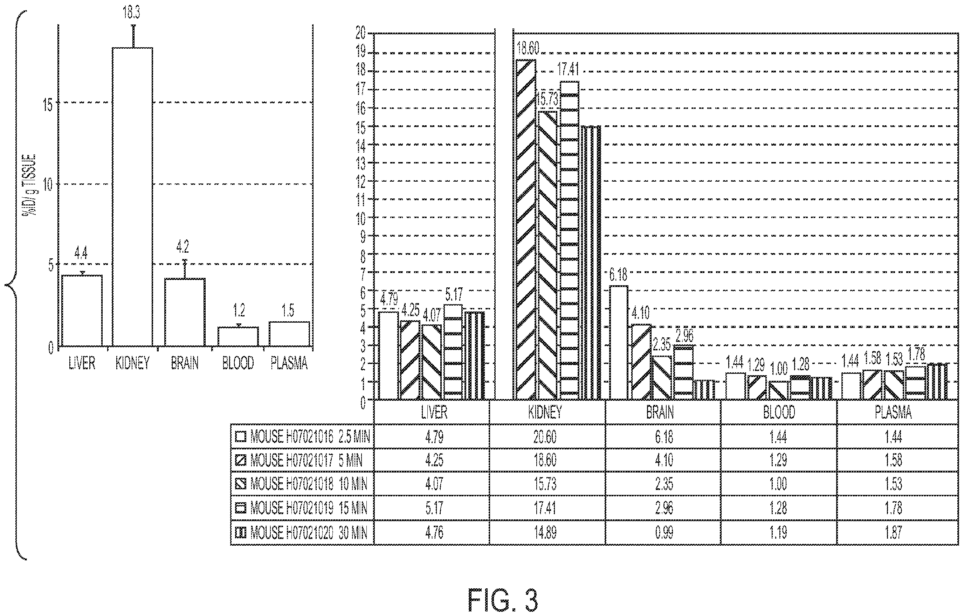

Figure 3 shows [18F]-T794 PK in mice.
Figure 4 shows Audoradiography of [18F]-T805.
Figure 5 shows the correlation of [18F]-T805 with Tau and Amyloid loads.
Figure 6 shows [18F]-T805 PK in mice.
Figure 7 shows Audoradiography of [18F]-T807.
Figure 8 shows the correlation of [18F]-T807 with Tau and Amyloid loads.
Figure 9 shows [18F]-T807 PK in mice.
Figure 10 shows double labeling of compound T687 and PHF-tau IHC staining on human brain section.
Figure 11 shows Double labeling of Compound T794 and total-tau IHC Staining on Human Brain Section.
Figure 12 shows 18F-T805: Brain uptake in mice.
Figure 13 shows 18F-T807: Brain uptake in mice.
Figure 14 shows 18F-T794 in WT and Tau mice.

### SUMMARY

Herein disclosed is a radiolabeled compound of the Formula 7: 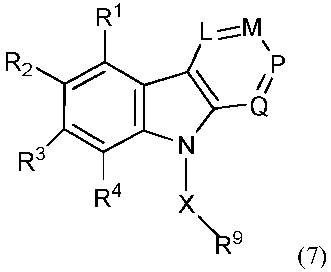 and pharmaceutically acceptable salts and stereoisomers thereof,
wherein:
L is N or CR⁵;
M is N or CR⁶;
P is N or CR⁷; and
Q is N or CR⁸;
X is a bond or is C₁₋₁₄alkyl, wherein at least one carbon is optionally replaced by C(O), O, S, SO₂, or NH, NH-C₁₋₈alkyl, and wherein at least one H of C₁₋₈alkyl is optionally replaced by halo, OH, C₁₋₆alkyl;
R⁹ is H, a protecting group, a leaving group, an azide, an alkyne, OH, halo, NH₂, N(C₁₋₈alkyl)₂, aryl or heteroaryl, wherein at least one H of the aryl or heteroaryl is optionally replaced by halo, NH₂, or C₁₋₆ alkyl, wherein at least one H of the C₁₋₆ alkyl is optionally replaced by halo, or C₃₋₈cycloalkyl, wherein at least one H of the C₃₋₈cycloalkyl is optionally replaced by halo and wherein at least one CH₂ of the C₃₋₈cycloalkyl is optionally replaced with O, OH, S, SH, NH, N-C₁₋₈alkyl;
R¹-R⁸ are independently H, OH, halo, NH₂, CH₃, NO₂, a leaving group, a protecting group, aryl, heteroaryl, NHR¹², N(R¹²)₂ C₃₋₈cycloalkyl, (-CH₂)₁₋₁₂-R¹², wherein R¹² is CH₃, aryl, H or heteroaryl,
wherein at least one H of (-CH₂)₁₋₁₂-R¹², C₃₋₈cycloalkyl, aryl, or heteroaryl, is optionally replaced by halo, OH, NH₂, a leaving group, a protecting group and C₁₋₈alkyl, wherein at least one H of the C₁₋₈alkyl is optionally replaced by halo, OH, NH₂, a leaving group, a protecting group, and
wherein at least one CH₂ of (-CH₂)₁₋₁₂-R¹² is optionally replaced with C(O), O, S, SO₂, or NH, NH-C₁₋₈alkyl, N(C₁₋₈alkyl)₂, wherein at least one H of the C₁₋₈alkyl is optionally replaced by halo, OH, NH₂, a leaving group, a protecting group,
and wherein at least one CH₂ of the C₃₋₈cycloalkyl is optionally replaced by C(O), O, S or NH, N-C₁₋₈alkyl, wherein at least one H of the C₁₋₈alkyl is optionally replaced by halo, OH, a leaving group, a protecting group,
wherein at least one halo is optionally replaced with a radionuclide or a fluorescent tag.

Herein disclosed is a compound of the Formula 7a: 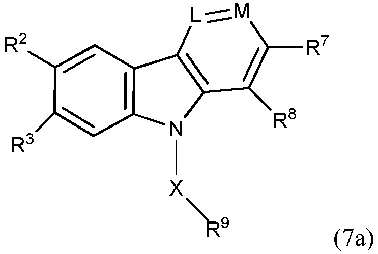 and pharmaceutically acceptable salts and stereoisomers thereof,
wherein:
L is N or CR⁵;
M is N or CR⁶;
X is a bond or is C₁₋₁₄alkyl, wherein at least one carbon is optionally replaced by C(O), O, S, SO₂, or NH, NH-C₁₋₈alkyl, and wherein at least one H of C₁₋₈alkyl is optionally replaced by halo, OH, C₁₋₆alkyl;
R⁹ is H, a protecting group, a leaving group, an azide, an alkyne, OH, halo, NH₂, N(C₁₋₈alkyl)₂, aryl or heteroaryl, wherein at least one H of the aryl or heteroaryl is optionally replaced by halo, NH₂, or C₁₋₆ alkyl or C₁₋₆ alkyl, wherein at least one H of the C₁₋₆ alkyl is optionally replaced by halo, or C₃₋₈cycloalkyl, wherein at least one H of the C₃₋₈cycloalkyl is optionally replaced by halo and wherein at least one CH₂ of the C₃₋₈cycloalkyl is optionally replaced with O, OH, S, SH, NH, N-C₁₋₈alkyl;
R², R³, R⁷ and R⁸ are independently H, OH, halo, NH₂, CH₃, NO₂, a leaving group, a protecting group, aryl, heteroaryl, NHR¹², N(R¹²)₂ C₃₋₈cycloalkyl, (-CH₂)₁₋₁₂-R¹², wherein R¹² is CH₃, aryl, H or heteroaryl,
wherein at least one H of (-CH₂)₁₋₁₂-R¹², C₃₋₈cycloalkyl, aryl, or heteroaryl, is optionally replaced by halo, OH, NH₂, a leaving group, a protecting group and C₁₋₈alkyl, wherein at least one H of the C₁₋₈alkyl is optionally replaced by halo, OH, NH₂, a leaving group, a protecting group, and
wherein at least one CH₂ of (-CH₂)₁₋₁₂-R¹² is optionally replaced with C(O), O, S, SO₂, or NH, NH-C₁₋₈alkyl, N(C₁₋₈alkyl)₂, wherein at least one H of the C₁₋₈alkyl is optionally replaced by halo, OH, NH₂, a leaving group, a protecting group,
and wherein at least one CH₂ of the C₃₋₈cycloalkyl is optionally replaced by C(O), O, S or NH, N-C₁₋₈alkyl, wherein at least one H of the C₁₋₈alkyl is optionally replaced by halo, OH, a leaving group, a protecting group,
wherein at least one halo is optionally replaced with a radionuclide or a fluorescent tag.

In one embodiment, the invention is a compound of the Formula 8: 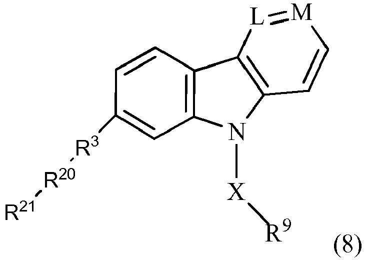 and pharmaceutically acceptable salts and stereoisomers thereof,
wherein:
L is N or CR⁵;
M is N;
R⁵ is H, OH, halo, NH₂, CH₃, NO₂, aryl, C₃-C₈alkenyl, C₃-C₈alkynyl, C₃₋₈cycloalkyl, heteroaryl, N=NR¹², NHR¹², N(R¹²)₂, or (-CH₂)₁₋₁₂-R¹², wherein R¹² is C₁-C₆alkyl, aryl, H or heteroaryl,
wherein at least one H of (-CH₂)₁₋₁₂-R¹², C₃₋₈cycloalkyl, aryl, alkenyl, alkynyl, or heteroaryl, is optionally replaced by halo, OH, NH₂, and C₁₋₈alkyl, wherein at least one H of the C₁₋₈alkyl is optionally replaced by halo, OH or NH₂, and
wherein at least one CH₂ of (-CH₂)₁₋₁₂-R¹² is optionally replaced with C(O), O, S, SO₂, or NH, NH-C₁₋₈alkyl or N(C₁₋₈alkyl)₂, wherein at least one H of the C₁₋₈alkyl is optionally replaced by halo, OH or NH₂,
and wherein at least one CH₂ of the C₃₋₈cycloalkyl is optionally replaced by C(O), O, S, NH or N-C₁₋₈alkyl, wherein at least one H of the C₁₋₈alkyl is optionally replaced by halo or OH,
X is a bond or is C₁₋₁₄alkyl, wherein at least one carbon is optionally replaced by C(O), O, S, SO₂, or NH, NH-C₁₋₈alkyl, and wherein at least one H of C₁₋₈alkyl is optionally replaced by halo, OH, C₁₋₆alkyl;
R⁹ is H, an azide, an alkyne, OH, halo, NH₂, N(C₁₋₈alkyl)₂, aryl or heteroaryl, wherein at least one H of the aryl or heteroaryl is optionally replaced by halo, NH₂, or C₁₋₆ alkyl or C₁₋₆ alkyl, wherein at least one H of the C₁₋₆ alkyl is optionally replaced by halo, or C₃₋₈cycloalkyl, wherein at least one H of the C₃₋₈cycloalkyl is optionally replaced by halo and wherein at least one CH₂ of the C₃₋₈cycloalkyl is optionally replaced with O, OH, S, SH, NH, N-C₁₋₈alkyl;
R³ is a bond or is at least one of O, S, C(O), SO₂, NH, N-C₁₋₈alkyl, (CH₂)₁₋₁₂, wherein at least one C of (CH₂)₁₋₁₂ is optionally replaced by C(O), O, S, SO₂, NH, N-C₁₋₈alkyl and wherein at least one H is optionally replaced by C₁₋₈alkyl or halo,
R²⁰ is aryl or heteroaryl; and
R²¹ is H, OH, halo, NH₂, CH₃, NO₂, (-CH₂)₁₋₁₂-CH₃, or C₃₋₈cycloalkyl,
wherein at least one H of the (-CH₂)₁₋₁₂-CH₃ or the C₃₋₈cycloalkyl is optionally replaced by halo, OH, NH₂, or C₁₋₈alkyl, wherein at least one H of the C₁₋₈alkyl is optionally replaced by halo orOH,
and wherein at least one CH₂ of the (-CH₂)₁₋₁₂-CH₃ is optionally replaced with C(O), O, S, SO₂, or NH, NH-C₁₋₈alkyl, N(C₁₋₈alkyl)₂, wherein at least one H of the C₁₋₈alkyl is optionally replaced by halo, OH or NH₂,
and wherein at least one CH₂ of the C₃₋₈cycloalkyl is optionally replaced by C(O), O, S, SO₂, or NH, N-C₁₋₈alkyl, wherein at least one H of the C₁₋₈alkyl is optionally replaced by halo, OH or NH₂,
and wherein R²¹ comprises a radionuclide,
provided that the compound is not

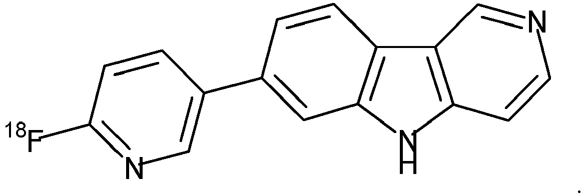

Herein disclosed is a compound of the Formula 7b: 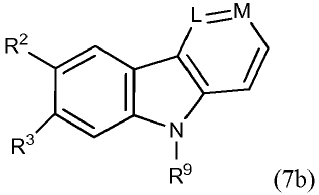 and pharmaceutically acceptable salts and stereoisomers thereof,
wherein:
L is N or CR⁵;
M is N or CR⁶;
R⁹ is H, a protecting group, a leaving group, halo, or CH₃;
R², R³, R⁵ and R⁶ are independently H, OH, halo, NH₂, CH₃, NO₂, a leaving group, a protecting group, aryl, heteroaryl, NHR¹², N(R¹²)₂ C₃₋₈cycloalkyl, (-CH₂)₁₋₁₂-R¹², wherein R¹² is CH₃, aryl, H or heteroaryl,
wherein at least one H of (-CH₂)₁₋₁₂-R¹², C₃₋₈cycloalkyl, aryl, or heteroaryl, is optionally replaced by halo, OH, NH₂, a leaving group, a protecting group and C₁₋₈alkyl, wherein at least one H of the C₁₋₈alkyl is optionally replaced by halo, OH, NH₂, a leaving group, a protecting group, and
wherein at least one CH₂ of (-CH₂)₁₋₁₂-R¹² is optionally replaced with C(O), O, S, SO₂, or NH, NH-C₁₋₈alkyl, N(C₁₋₈alkyl)₂, wherein at least one H of the C₁₋₈alkyl is optionally replaced by halo, OH, NH₂, a leaving group, a protecting group,
and wherein at least one CH₂ of the C₃₋₈cycloalkyl is optionally replaced by C(O), O, S or NH, N-C₁₋₈alkyl, wherein at least one H of the C₁₋₈alkyl is optionally replaced by halo, OH, a leaving group, a protecting group,
wherein at least one halo is optionally replaced with a radionuclide or a fluorescent tag.

Herein disclosed is a compound of the Formula 7c: 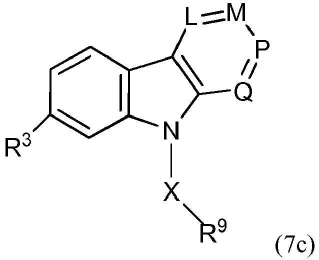 and pharmaceutically acceptable salts and stereoisomers thereof,
wherein:
L is N or CR⁵;
M is N or CR⁶;
P is N or CR⁷; and
Q is N or CR⁸;
X is a bond or is C₁₋₁₄alkyl, wherein at least one carbon is optionally replaced by C(O), O, S, SO₂, or NH, NH-C₁₋₈alkyl, and wherein at least one H of C₁₋₈alkyl is optionally replaced by halo, OH, C₁₋₆alkyl;
R⁹ is H, a protecting group, a leaving group, OH, NH₂, N(C₁₋₈alkyl)₂, aryl or heteroaryl, wherein at least one H of the aryl or heteroaryl is optionally replaced by NH₂, or C₁₋₆ alkyl, wherein at least one H of the C₁₋₆ alkyl is optionally replaced by C₃₋₈cycloalkyl, wherein at least one CH₂ of the C₃₋₈cycloalkyl is optionally replaced with O, OH, S, SH, NH, N-C₁₋₈alkyl;
R³ and R⁵-R⁸ are independently H or (-CH₂)₁₋₁₂-R¹³, wherein R¹³ is an azide or an alkyne,
wherein at least one H of (-CH₂)₁₋₁₂-R¹³ is optionally replaced by OH, NH₂, and C₁₋₈alkyl, wherein at least one H of the C₁₋₈alkyl is optionally replaced by OH, NH₂, and
wherein at least one CH₂ of (-CH₂)₁₋₁₂-R¹³ is optionally replaced with C(O), O, S, SO₂, or NH, NH-C₁₋₈alkyl, N(C₁₋₈alkyl)₂, wherein at least one H of the C₁₋₈alkyl is optionally replaced by OH, NH₂.

Also disclosed herein is a pharmaceutical composition for in vivo imaging of amyloid deposits and tau tangles, comprising (a) the compound of any of the Formulas herein and/or shown in Claims 1-7 and (b) a pharmaceutically acceptable carrier.

Also disclosed herein is a method of diagnosing Alzheimer's Disease or a predisposition thereto in a mammal, the method comprising: a) administering to the mammal a diagnostically effective amount of a radiolabeled compound of any of the Formulas herein, wherein the compound passes the blood-brain barrier and preferentially binds to amyloid plaques and/or tau tangles in a brain tissue and wherein the compound is selected from the group consisting of radiolabeled compounds of formula 8, for example; b) allowing the compound to distribute into the brain tissue; and c) imaging the brain tissue, wherein an increase in binding of the compound to the brain tissue compared to a normal control level of binding indicates that the mammal is suffering from or is at risk of developing Alzheimer's Disease.

Also disclosed herein is a method of diagnosing Alzheimer's Disease or a predisposition thereto in a mammal, the method comprising: a) administering to the mammal a diagnostically effective amount of a radiolabeled compound of any of Claims 1-7, wherein the compound passes the blood-brain barrier and preferentially binds to amyloid plaques and/or tau tangles in a brain tissue and wherein the compound is selected from the group consisting of radiolabeled compounds of formula 8; b) allowing the compound to distribute into the brain tissue; and c) imaging the brain tissue, wherein an increase in binding of the compound to the brain tissue compared to a normal control level of binding indicates that the mammal is suffering from or is at risk of developing Alzheimer's Disease.

### DETAILED DESCRIPTION

"Halogen" or "halo" means F, Cl, Br and I.

"Alkyl" means a saturated monovalent hydrocarbon radical having straight or branched moieties. Examples of alkyl groups include, but are not limited to, methyl, ethyl, n-propyl, isopropyl and t-butyl.

"Alkenyl" means an alkyl moieties having at least one carbon-carbon double bond wherein alkyl is as defined above. Examples of alkenyl include, but are not limited to, ethenyl and propenyl.

"Alkynyl" means alkyl moieties having at least one carbon-carbon triple bond wherein alkyl is as defined above. Examples of alkynyl groups include, but are not limited to, ethynyl and 2-propynyl.

"Alkylene" or "alkenylenyl" means a saturated, divalent hydrocarbon radicals i.e., generally present as a bridging or linking group between two other groups, having straight or branched moieties. Examples of alkylene groups include -CH₂-(methylene); - CH₂CH₂-(ethylene); -CH₂CH₂CH₂-(propylene), -CH(CH₃)CH₂-(isopropylene) etc.

"Amino" means a nitrogen moiety having two further substituents where a hydrogen or carbon atom is attached to the nitrogen. For example, representative amino groups include -NH₂, -NHCH₃, -N(CH₃)₂, -NHC₂₋₃-alkyl, -N(C₂₋₃-alkyl)₂ and the like. Unless indicated otherwise, the compounds of the invention containing amino moieties may include protected derivatives thereof. Suitable protecting groups for amino moieties include acetyl, *tert*-butoxycarbonyl, benzyloxycarbonyl and the like.

"Aryl" means an organic radical derived from an aromatic hydrocarbon by removal of one hydrogen, such as phenyl, naphthyl, indenyl, indanyl and fluorenyl. "Aryl" encompasses fused ring groups wherein at least one ring is aromatic.

"Cycloalkyl" means non-aromatic saturated cyclic alkyl moieties consisting of one or more rings, wherein said rings (if more than one) share at least one carbon atom, wherein alkyl is as defined above. Examples of cycloalkyl include, but are not limited to, cyclopropyl, cyclobutyl, cyclopentyl, cyclohexyl, cycloheptyl, bicyclo-[3.1.0]-hexyl, bicyclo-[2.2.1]-hept-1-yl, norbornyl, spiro[4.5]decyl, spiro[4.4]nonyl, spiro[4.3]octyl, spiro[4.2]heptyl and adamantanyl.

"HaloC₁-6alkyl" means a C₁-6alkyl group that is substituted with at least one halogen atom on a carbon atom of the alkyl group. Non-exclusive, representative examples of such haloC₁-₆alkyl include F-CH₂-, F-CH₂CH₂-, F-CH₂CH₂CH₂-, CHF₂-, CHF₂CH₂-, CHF₂CH₂CH₂-, Br-CH₂-, Br-CH₂CH₂-, Br-CH₂CH₂CH₂-, CHBr₂-, CHBr₂CH₂-, CHBr₂CH₂CH₂- and the like.

"Heterocyclic" or "heterocycloalkyl" means a non-aromatic cyclic groups consisting of one or more rings, wherein the rings (if more than one) share one or two atoms and each ring contains up to four heteroatoms (i.e. from zero to four heteroatoms, provided that at least one ring contains at least one heteroatom). The heterocyclic groups of this invention can also include ring systems substituted with one or more O, S(O)₀₋₂, and/or N-R¹⁰ as heteroatoms, wherein R¹⁰ is as defined herein, and wherein the subscript "0-2" of S(O)₀₋₂ represents an integer of 0, 1 or 2. Thus, S(O)₀₋₂ represents the group consisting of S, S(=O), and S(O)₂. Examples of non-aromatic heterocyclic groups are aziridinyl, azetidinyl, pyrrolidinyl, piperidinyl, azepinyl, piperazinyl, 1,2,3,6-tetrahydropyridinyl, oxiranyl, oxetanyl, tetrahydrofuranyl, tetrahydrothienyl, tetrahydropyranyl, tetrahydrothiopyranyl, morpholino, thiomorpholino, thioxanyl, pyrrolinyl, indolinyl, 2H-pyranyl, 4H-pyranyl, dioxanyl, 1,3-dioxolanyl, pyrazolinyl, dihydropyranyl, dihydrothienyl, dihydrofuranyl, pyrazolidinyl, imidazolinyl, imidazolidinyl, 3-azabicyclo[3.1.0]hexanyl, 3-azabicyclo[4.1.0]heptanyl, quinolizinyl, quinuclidinyl, 1,4-dioxaspiro[4.5]decyl, 1,4-dioxaspiro[4.4]nonyl, 1,4-dioxaspiro[4.3]octyl and 1,4-dioxaspiro[4.2]heptyl.

"Heteroaryl" means an aromatic group containing one or more heteroatoms (O, S, or N), preferably from one to four heteroatoms. A heteroaryl may be a monocyclic or a polycyclic group. Examples of heteroaryl groups are pyridinyl, pyridazinyl, imidazolyl, pyrimidinyl, pyrazolyl, triazolyl, pyrazinyl, quinolyl, isoquinolyl, 1,2,3,4-tetrahydroguinolyl, tetrazolyl, furyl, thienyl, isoxazolyl, thiazolyl, oxazolyl, isothiazolyl, pyrrolyl, indolyl, benzimidazolyl, benzofuranyl, indazolyl, indolizinyl, phthalazinyl, triazinyl, 1,3,5-triazinyl, isoindolyl, purinyl, oxadiazolyl, thiadiazolyl, furazanyl, benzofurazanyl, benzothiophenyl, benzotriazolyl, benzothiazolyl, benzoxazolyl, quinazolinyl, quinoxalinyl, naphthyridinyl, dihydroquinolyl, tetrahydroquinolyl, dihydroisoquinolyl, tetrahydroisoquinolyl, benzofuryl, furopyridinyl, pyrolopyrimidinyl and azaindolyl. In certain aspects of the present application, the heteroaryl is a 4-substituted-1H-1,2-3-triazol-1-yl.

As used herein, where a divalent group, such as a linker for example, is represented by a structure -A-B-, as shown below, it is intended to also represent a group that may be attached in both possible permutations, as noted in the two structures below.

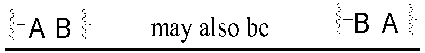

For example, when a divalent group such as the group "-N(R¹⁰)C(O)-" is provided, for example, the group is intended to also include both the divalent group -N(R¹⁰)C(O)- and also the divalent group -C(O)N(R¹⁰)-.

The substituents or the groups C₁₋₆alkyl, C₃₋₆cycloalkyl, C₃₋₁₂cycloalkylC₁₋₅alkyl, C₆₋₁₄aryl, C₆₋₁₄aryloxy, C₆₋₁₀arylC₁₋4alkyl, heteroaryl, heteroaryloxy etc ... of the variables R¹, R², R³, R⁴, R⁵, R⁶, R⁷, R⁸, R⁹ and R¹⁰ are also optionally further substituted by substituents selected from the group consisting of amino, halo, cyano, nitro, hydroxyl, -SH, -SC₁₋₆alkyl, -C(O)NH₂, -C(S)NH₂, haloC₁-₆alkyl, perhaloC₁-₆alkyl, C₁₋₆alkyl, C₃₋₆cycloalkyl, C₃₋₁₂cycloalkyl, C₆₋₁₄aryl and heteroaryl.

For example, the heteroaryl substituent may be a 4-substituted-1H-1,2-3-triazol-1-yl and a radionuclide may be attached to an aryl group of the compound of Formula I, as in a 2-¹⁸F-'carbazole derivative such as the compound represented as: 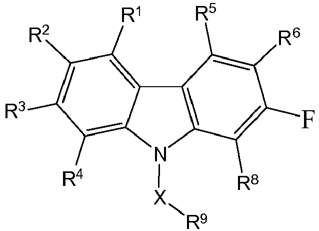 or a 2-(¹⁸F-fluoroethyl)-'carbazole, 2-(¹⁸F-fluoromethyl)-'carbazole, a ¹¹C-methoxy-group, for example, and/or the radionuclide may be attached to any one or more of the variables R¹, R², R³, R⁴, R⁵, R⁶, R⁷, R⁸, R⁹ and R¹⁰ by way of a ¹⁸F-fluoroethyl- group, a ¹⁸F-fluoromethyl- group, a ¹¹C-methoxy- group, 4-[(¹⁸F-fluoroethyl)-1H-1,2-3-triazol-1-yl]-ethoxy- group, 4-[(¹⁸F-fluoroethyl)-1H-1,2-3-triazol-1-yl]-propyloxy- group, a ¹²³I, a ¹²⁴I, a ¹²⁵I or a ¹³¹I group, and the like. Unless otherwise noted, a compound represented as being substituted by an atom, such as the generic representation by the atom fluorine in F-CH₂CH₂-('carbazole) or F-CH₂CH₂O-('carbazole), for example, is intended to cover both the naturally occurring element ¹⁹F (fluorine-19) as well as the ¹⁸F (fluorine-18) isotope(s) of the element itself.

The term "optionally substituted" or "substituted" refers to the specific substituents or groups wherein one to four hydrogen atoms in the group may be replaced by one to four substituents, for example, independently selected from the substituents amino, halo, cyano, nitro, hydroxyl, -SH, -SC₁₋₆alkyl, -C(O)NH₂, -C(S)NH₂, haloC₁-₆alkyl, perhaloC₁-₆alkyl, C₁₋₆alkyl, C₃₋₆cycloalkyl, C₃₋₁₂cycloalkyl, C₆₋₁₄aryl and heteroaryl, or as specifically disclosed herein. In addition, the substituents may also include alkyl, aryl, alkylene-aryl, hydroxy, alkoxy, aryloxy, perhaloalkoxy, heterocyclyl, azido, amino, guanidino, amidino, halo, alkylthio, oxo, acylalkyl, carboxy esters, carboxyl, carboxamido, acyloxy, aminoalkyl, alkylaminoaryl, alkylaminoalkyl, alkoxyaryl, arylamino, phosphono, sulfonyl, carboxamidoaryl, hydroxyalkyl, haloalkyl, alkoxyalkyl and perhaloalkyl. In addition, the term "optionally substituted" or "substituted" in reference to the variables R¹, R², R³, R⁴, R⁵, R⁶, R⁷, R⁸, R⁹ and R¹⁰, includes groups substituted by one to four substituents, as identified above, that further comprise a positron or gamma emitter. Such positron emitters include, but are not limited to, ¹¹C, ¹³N, ¹⁵O, ¹⁸F, ¹²³I, ¹²⁴I, ¹²⁵I, ¹³¹I and ⁷⁷Br.

The term "radiolabeled compound" as used herein refers to compounds having an atom or group that may provide a radiolabel or may be converted to a radio label, such as from a non-radioactive atom to a radionuclide that is active, such as for example, ¹¹C, ¹³N, ¹⁵O, ¹⁸F, ¹²³I, ¹²⁴I, ¹²⁵I, ¹³¹I and ⁷⁷Br. In addition, for the purpose of the present application, such "radiolabeled compound" may also refer to an atom or a group, that comprises a non-active nuclide, such as a halogen, such as ¹⁹F for example, wherein the compound may be used and administered in a therapeutically effective amount.

Compounds of the Formulas disclosed herein may have optical centers and therefore may occur in different enantiomeric and diastereomeric configurations. The present invention includes all enantiomers, diastereomers, and other stereoisomers of such compounds of the Formulas disclosed herein, as well as racemic compounds and racemic mixtures and other mixtures of stereoisomers thereof. Pharmaceutically acceptable salts of the compounds of the Formulas disclosed herein include the acid addition and base salts thereof. Suitable acid addition salts are formed from acids which form non-toxic salts. Examples include, but are not limited to, the acetate, adipate, aspartate, benzoate, besylate, bicarbonate/carbonate, bisulphate/sulphate, borate, citrate, formate, fumarate, gluconate, glucuronate, hydrochloride/chloride, hydrobromide/bromide, hydroiodide/iodide, lactate, malate, maleate, malonate, mesylate, methylsulphate, naphthylate, oxalate, palmitate, phosphate/hydrogen phosphate/dihydrogen phosphate, pyroglutamate, salicylate, stearate, succinate, sulfonate, tartrate, tosylate and trifluoroacetate salts. Suitable base salts are formed from bases which form non-toxic salts. Examples include, but are not limited to, the aluminum, arginine, benzathine, calcium, choline, diethylamine, diolamine, glycine, lysine, magnesium, potassium, sodium, tromethamine and zinc salts. Hemisalts of acids and bases may also be formed, for example, hemisulphate and hemicalcium salts. For a review on suitable salts, see Handbook of Pharmaceutical Salts: Properties, Selection, and Use by Stahl and Wermuth (Wiley-VCH, 2002). Pharmaceutically acceptable salts of compounds of the Formulas disclosed herein may be prepared by one or more of three methods: (i) by reacting the compound of the Formulas disclosed herein with the desired acid or base; (ii) by removing an acid- or base-labile protecting group from a suitable precursor of the compound of the Formulas disclosed herein; or (iii) by converting one salt of the compound of the Formulas disclosed herein to another salt by the reaction with an appropriate acid or base or by means of a suitable ion exchange column.

Also disclosed herein is the imaging by employing a fluorescence imaging technique or a nuclear imaging technique selected from the group consisting of positron emission tomography (PET) and single photon emission computed tomography (SPECT), the fluorescence imaging technique and/or nuclear imaging technique for monitoring or visualizing a distribution of the radiolabeled or tagged compound within the brain or within a portion thereof.

Also disclosed herein is a compound of formula 8 for use in the treatment of a disease or condition, in a mammal in need thereof, selected from the group consisting of anxiety, depression, schizophrenia, Alzheimer's Disease, stress-related disease, panic, a phobia, obsessive compulsive disorder, obesity, post-traumatic stress syndrome, or epilepsy.

Herein disclosed is a radiolabeled compound of the formula: 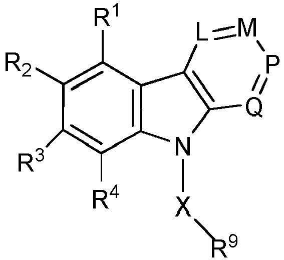 and pharmaceutically acceptable salts and stereoisomers thereof,
wherein:
L is N or CR⁵;
M is N or CR⁶;
P is N or CR⁷; and
Q is N or CR⁸;
X is a bond or is C₁₋₁₄alkyl, wherein at least one carbon is optionally replaced by C(O), O, S, SO₂, or NH, NH-C₁₋₈alkyl, and wherein at least one H of C₁₋₈alkyl is optionally replaced by halo, OH, C₁₋₆alkyl;
R⁹ is H, a protecting group, a leaving group, an azide, an alkyne, OH, halo, NH₂, N(C₁₋₈alkyl)₂, aryl or heteroaryl, wherein at least one H of the aryl or heteroaryl is optionally replaced by halo, NH₂, or C₁₋₆ alkyl, wherein at least one H of the C₁₋₆ alkyl is optionally replaced by halo, or C₃₋₈cycloalkyl, wherein at least one H of the C₃₋₈cycloalkyl is optionally replaced by halo and wherein at least one CH₂ of the C₃₋₈cycloalkyl is optionally replaced with O, OH, S, SH, NH, N-C₁-₈alkyl;
R¹-R⁸ are independently H, OH, halo, NH₂, CH₃, a leaving group, a protecting group, aryl, heteroaryl, NHR¹², N(R¹²)₂ C₃₋₈cycloalkyl, (-CH₂)₁₋₁₂-R¹², wherein R¹² is CH₃, aryl, H or heteroaryl,
wherein at least one H of (-CH₂)₁₋₁₂-R¹², C₃₋₈cycloalkyl, aryl, or heteroaryl, is optionally replaced by halo, OH, NH₂, a leaving group, a protecting group and C₁₋₈alkyl, wherein at least one H of the C₁₋₈alkyl is optionally replaced by halo, OH, NH₂, a leaving group, a protecting group, and
wherein at least one CH₂ of (-CH₂)₁₋₁₂-R¹² is optionally replaced with C(O), O, S, SO₂, or NH, NH-C₁₋₈alkyl, N(C₁₋₈alkyl)₂, wherein at least one H of the C₁₋₈alkyl is optionally replaced by halo, OH, NH₂, a leaving group, a protecting group,
and wherein at least one CH₂ of the C₃₋₈cycloalkyl is optionally replaced by C(O), O, S or NH, N-C₁₋₈alkyl, wherein at least one H of the C₁₋₈alkyl is optionally replaced by halo, OH, a leaving group, a protecting group,
wherein at least one halo is optionally replaced with a radionuclide or a fluorescent tag.

Herein disclosed is a compound of the formula: 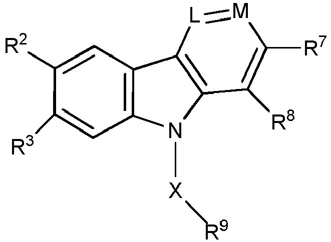 and pharmaceutically acceptable salts and stereoisomers thereof,
wherein:
L is N or CR⁵;
M is N or CR⁶;
X is a bond or is C₁₋₁₄alkyl, wherein at least one carbon is optionally replaced by C(O), O, S, SO₂, or NH, NH-C₁₋₈alkyl, and wherein at least one H of C₁₋₈alkyl is optionally replaced by halo, OH, C₁₋₆alkyl;
R⁹ is H, a protecting group, a leaving group, an azide, an alkyne, OH, halo, NH₂, N(C₁₋₈alkyl)₂, aryl or heteroaryl, wherein at least one H of the aryl or heteroaryl is optionally replaced by halo, NH₂, or C₁₋₆ alkyl or C₁₋₆ alkyl, wherein at least one H of the C₁₋₆ alkyl is optionally replaced by halo, or C₃₋₈cycloalkyl, wherein at least one H of the C₃₋₈cycloalkyl is optionally replaced by halo and wherein at least one CH₂ of the C₃₋₈cycloalkyl is optionally replaced with O, OH, S, SH, NH, N-C₁₋₈alkyl;
R², R³, R⁷ and R⁸ are independently H, OH, halo, NH₂, CH₃, a leaving group, a protecting group, aryl, heteroaryl, NHR¹², N(R¹²)₂ C₃₋₈cycloalkyl, (-CH₂)₁₋₁₂-R¹², wherein R¹² is CH₃, aryl, H or heteroaryl,
wherein at least one H of (-CH₂)₁₋₁₂-R¹², C₃₋₈cycloalkyl, aryl, or heteroaryl, is optionally replaced by halo, OH, NH₂, a leaving group, a protecting group and C₁₋₈alkyl, wherein at least one H of the C₁₋₈alkyl is optionally replaced by halo, OH, NH₂, a leaving group, a protecting group, and
wherein at least one CH₂ of (-CH₂)₁₋₁₂-R¹² is optionally replaced with C(O), O, S, SO₂, or NH, NH-C₁₋₈alkyl, N(C₁₋₈alkyl)₂, wherein at least one H of the C₁₋₈alkyl is optionally replaced by halo, OH, NH₂, a leaving group, a protecting group,
and wherein at least one CH₂ of the C₃₋₈cycloalkyl is optionally replaced by C(O), O, S or NH, N-C₁₋₈alkyl, wherein at least one H of the C₁₋₈alkyl is optionally replaced by halo, OH, a leaving group, a protecting group,
wherein at least one halo is optionally replaced with a radionuclide or a fluorescent tag.

In another embodiment, the invention is a compound of the formula: 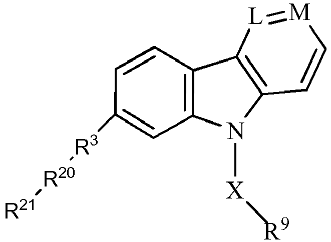 and pharmaceutically acceptable salts and stereoisomers thereof,
wherein:
L is N or CR⁵;
M is N or;
R⁵ is H, OH, halo, NH₂, CH₃, NO₂, aryl, C₃-C₈alkenyl, C₃-C₈alkynyl, C₃₋₈cycloalkyl, heteroaryl, N=NR¹², NHR¹², N(R¹²)₂, or (-CH₂)₁₋₁₂-R¹², wherein R¹² is C₁-C₆alkyl, aryl, H or heteroaryl,
wherein at least one H of (-CH₂)₁₋₁₂-R¹², C₃₋₈cycloalkyl, aryl, alkenyl, alkynyl, or heteroaryl, is optionally replaced by halo, OH, NH₂, and C₁₋₈alkyl, wherein at least one H of the C₁₋₈alkyl is optionally replaced by halo, OH or NH₂, and
wherein at least one CH₂ of (-CH₂)₁₋₁₂-R¹² is optionally replaced with C(O), O, S, SO₂, or NH, NH-C₁₋₈alkyl or N(C₁₋₈alkyl)₂, wherein at least one H of the C₁₋₈alkyl is optionally replaced by halo, OH or NH₂,
and wherein at least one CH₂ of the C₃₋₈cycloalkyl is optionally replaced by C(O), O, S, NH or N-C₁₋₈alkyl, wherein at least one H of the C₁₋₈alkyl is optionally replaced by halo or OH,
X is a bond or is C₁₋₁₄alkyl, wherein at least one carbon is optionally replaced by C(O), O, S, SO₂, or NH, NH-C₁₋₈alkyl, and wherein at least one H of C₁₋₈alkyl is optionally replaced by halo, OH, C₁₋₆alkyl;
R⁹ is H, an azide, an alkyne, OH, halo, NH₂, N(C₁₋₈alkyl)₂, aryl or heteroaryl, wherein at least one H of the aryl or heteroaryl is optionally replaced by halo, NH₂, or C₁₋₆ alkyl or C₁₋₆ alkyl, wherein at least one H of the C₁₋₆ alkyl is optionally replaced by halo, or C₃₋₈cycloalkyl, wherein at least one H of the C₃₋₈cycloalkyl is optionally replaced by halo and wherein at least one CH₂ of the C₃₋₈cycloalkyl is optionally replaced with O, OH, S, SH, NH, N-C₁₋₈alkyl;
R³ is a bond or is at least one of O, S, C(O), SO₂, NH, N-C₁₋₈alkyl, (CH₂)₁₋₁₂, wherein at least one C of (CH₂)₁₋₁₂ is optionally replaced by C(O), O, S, SO₂, NH, N-C₁₋₈alkyl and wherein at least one H is optionally replaced by C₁₋₈alkyl or halo,
R²⁰ is aryl or heteroaryl; and
R²¹ is H, OH, halo, NH₂, CH₃, NO₂, (-CH₂)₁₋₁₂-CH₃, C₃₋₈cycloalkyl,
wherein at least one H of the (-CH₂)₁₋₁₂-CH₃ or the C₃₋₈cycloalkyl is optionally replaced by halo, OH, NH₂ or C₁₋₈alkyl, wherein at least one H of the C₁₋₈alkyl is optionally replaced by halo or OH,
and wherein at least one CH₂ of the (-CH₂)₁₋₁₂-CH₃ is optionally replaced with C(O), O, S, SO₂, or NH, NH-C₁₋₈alkyl, N(C₁₋₈alkyl)₂, wherein at least one H of the C₁₋₈alkyl is optionally replaced by halo, OH or NH₂,
and wherein at least one CH₂ of the C₃₋₈cycloalkyl is optionally replaced by C(O), O, S, SO₂, or NH, N-C₁₋₈alkyl, wherein at least one H of the C₁₋₈alkyl is optionally replaced by halo, OH or NH₂,
and wherein R²¹ comprises a radionuclide, provided that the compound is not

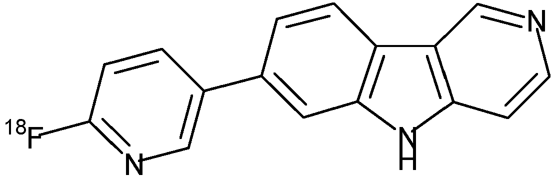

Herein disclosed is a compound of the formula: 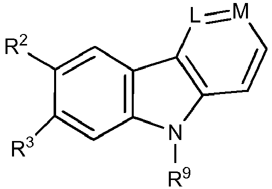 and pharmaceutically acceptable salts and stereoisomers thereof,
wherein:
L is N or CR⁵;
M is N or CR⁶;
R⁹ is H, a protecting group, a leaving group, halo, or CH₃;
R², R³, R⁵ and R⁶ are independently H, OH, halo, NH₂, CH₃, , a leaving group, a protecting group, aryl, heteroaryl, NHR¹², N(R¹²)₂ C₃₋₈cycloalkyl, (-CH₂)₁₋₁₂-R¹², wherein R¹² is CH₃, aryl, H or heteroaryl,
wherein at least one H of (-CH₂)₁₋₁₂-R¹², C₃₋₈cycloalkyl, aryl, or heteroaryl, is optionally replaced by halo, OH, NH₂, a leaving group, a protecting group and C₁₋₈alkyl, wherein at least one H of the C₁₋₈alkyl is optionally replaced by halo, OH, NH₂, a leaving group, a protecting group, and
wherein at least one CH₂ of (-CH₂)₁₋₁₂-R¹² is optionally replaced with C(O), O, S, SO₂, or NH, NH-C₁₋₈alkyl, N(C₁₋₈alkyl)₂, wherein at least one H of the C₁₋₈alkyl is optionally replaced by halo, OH, NH₂, a leaving group, a protecting group,
and wherein at least one CH₂ of the C₃₋₈cycloalkyl is optionally replaced by C(O), O, S or NH, N-C₁₋₈alkyl, wherein at least one H of the C₁₋₈alkyl is optionally replaced by halo, OH, a leaving group, a protecting group,
wherein at least one halo is optionally replaced with a radionuclide or a fluorescent tag.

Herein disclosed is a compound of the formula: 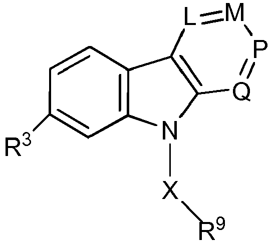 and pharmaceutically acceptable salts and stereoisomers thereof,
wherein:
L is N or CR⁵;
M is N or CR⁶;
P is N or CR⁷; and
Q is N or CR⁸;
X is a bond or is C₁₋₁₄alkyl, wherein at least one carbon is optionally replaced by C(O), O, S, SO₂, or NH, NH-C₁₋₈alkyl, and wherein at least one H of C₁₋₈alkyl is optionally replaced by halo, OH, C₁₋₆alkyl;
R⁹ is H, a protecting group, a leaving group, OH, NH₂, N(C₁₋₈alkyl)₂, aryl or heteroaryl, wherein at least one H of the aryl or heteroaryl is optionally replaced by SO₂, NH₂, or C₁₋₆ alkyl, wherein at least one H of the C₁₋₆ alkyl is optionally replaced by C₃₋₈cycloalkyl, wherein at least one CH₂ of the C₃₋₈cycloalkyl is optionally replaced with O, OH, S, SH, NH, N-C₁₋₈alkyl;
R³ and R⁵-R⁸ are independently H or (-CH₂)₁₋₁₂-R¹³, wherein R¹³ is an azide or an alkyne,
wherein at least one H of (-CH₂)₁₋₁₂-R¹³ is optionally replaced by OH, NH₂, and C₁₋₈alkyl, wherein at least one H of the C₁₋₈alkyl is optionally replaced by OH, NH₂, and
wherein at least one CH₂ of (-CH₂)₁₋₁₂-R¹³ is optionally replaced with C(O), O, S, SO₂, or NH, NH-C₁₋₈alkyl, N(C₁₋₈alkyl)₂, wherein at least one H of the C₁₋₈alkyl is optionally replaced by OH, NH₂.

Also disclosed herein is a pharmaceutical composition for in vivo imaging of amyloid deposits and tau tangles, comprising (a) the compound of any of the Formulas above or shown in Claims 1-7 and (b) a pharmaceutically acceptable carrier.

Also disclosed herein is a method of diagnosing Alzheimer's Disease or a predisposition thereto in a mammal, the method comprising: a) administering to the mammal a diagnostically effective amount of a radiolabeled compound, wherein the compound passes the blood-brain barrier and preferentially binds to amyloid plaques and/or tau tangles in a brain tissue and wherein the compound is selected from the group consisting of radiolabeled compounds of formula 8, for example; b) allowing the compound to distribute into the brain tissue; and c) imaging the brain tissue, wherein an increase in binding of the compound to the brain tissue compared to a normal control level of binding indicates that the mammal is suffering from or is at risk of developing Alzheimer's Disease.

Also disclosed herein is a method of diagnosing Alzheimer's Disease or a predisposition thereto in a mammal, the method comprising: a) administering to the mammal a diagnostically effective amount of a radiolabeled compound of any of Claims 1-7, provided below, wherein the compound passes the blood-brain barrier and preferentially binds to amyloid plaques and/or tau tangles in a brain tissue and wherein the compound is selected from the group consisting of radiolabeled compounds of formula 8, for example; b) allowing the compound to distribute into the brain tissue; and c) imaging the brain tissue, wherein an increase in binding of the compound to the brain tissue compared to a normal control level of binding indicates that the mammal is suffering from or is at risk of developing Alzheimer's Disease.

Also disclosed herein is a method for imaging and detection of senile plaques and/or neurofibrillary tangles in a brain tissue, the method comprising treating the tissue with a compound of formula8 for detection of neurological disorders.

The neurological disorder may be detected by measuring the affinity of compounds of formula8 for tau aggrerates.

In one embodiment, the detection may be by gamma imaging with PET or SPECT.

### Table 1

**Table 1: Known AD positive fluorescent dyes and imaging agents**

| **Name** | **Compound and Reference** | **Target** | **Binding Affinity** |
|---|---|---|---|
| Congo Red | 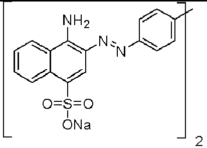 | Aβ monomer | IC₅₀: 2-10 uM |
| | Anal. Biochem. 2006, 356, 265-272; J. Biol. Chem. 2005, 280, 5892-5901 | | |
| Curcumin | 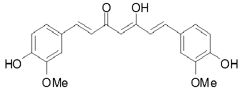 | Aβ monomer | IC₅₀: 10-20 uM |
| | Anal. Biochem. 2006, 356, 265-272; J. Biol. Chem. 2005, 280, 5892-5901 | | |
| ANS | 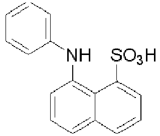 | Aβ monomer | IC₅₀: >100 uM |
| | Anal. Biochem. 2006, 356, 265-272 | | |
| Thioflavin T | 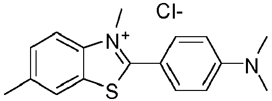 | Aβ monomer | IC₅₀: >500 uM |
| | Anal. Biochem. 2006, 356, 265-272 | | |
| Iodinated Flavone | 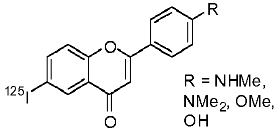 | Aβ40 aggregates | Ki = 13 nM (-NMe2) to 72 nM (-OH) |
| | J. Med. Chem. 2005,48, 7253-7260 | | |
| Pyridyl Styrene | 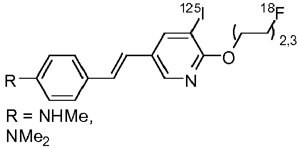 | Aβ fibrils | Kd = 7.5-9 nM |
| | J. Med. Chem. 2007,50, 2157-2165 | | |
| Diaryl acetylenes | 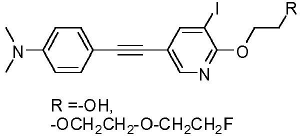 | Aβ plaques | Kd = ∼10 nM |
| | Bioorg. Med. Chem. 2007, 17, 3581-3584 | | |
| Thiophene chalcones | 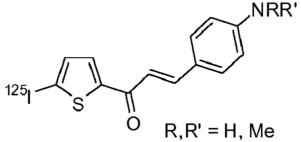 | Aβ 1-42 aggregates | Ki = 3.9-14 nM |
| | Bioorg. Med. Chem. 2007, 15, 6802-6809 | | |
| Aurones | 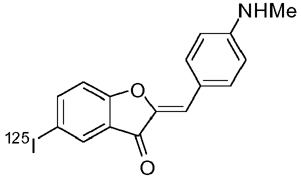 | Aβ 1-42 aggregates | Ki = 1.24 nM |
| | Biochem. Biophys. Res. Commun. 2007, 361, 116-121 | | |
| Benzofuran | 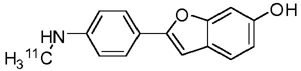 | Aβ fibrils | Ki = 2.8 nM |
| | J. Med. Chem. 2006,49, 2725-2730 | | |

**Table 2: The following are examples of compounds useful for detecting AD biomarkers in vivo and are included for reference purposes. These compounds may be radiolabeled or be "cold":**

| Name | Structure | Chemical Formula | MW | Code |
|---|---|---|---|---|
| 2-(2-fluoroethoxy)-9H-carbazole | 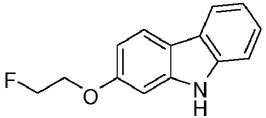 | C₁₄H₁₂FNO | 229.25 | CB-001 |
| 9-(2-fluoroethyl)-9H-carbazol-2-ol | 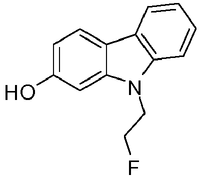 | C₁₄H₁₂FNO | 229.25 | |
| N-(2-fluoroethyl)-7-(2-(2-(2-methoxyethoxy)ethoxy)ethoxy)-9H-carbazol-3-amine | 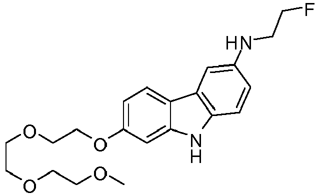 | C₂₁H₂₇FN₂O₄ | 390.45 | |
| 7-(2-fluoroethoxy)-N,N-dimethyl-9H-carbazol-2-amine | 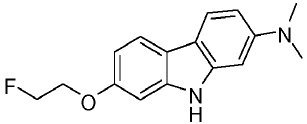 | C₁₆H₁₇FN₂O | 272.32 | |
| 7-(2-(2-(2-fluoroethoxy)ethoxy)ethoxy)-N-methyl-9H-carbazol-3-amine | 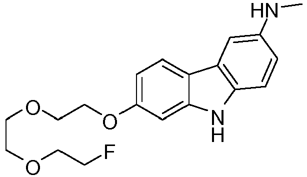 | C₁₉H₂₃FN₂O₃ | 346.40 | CB-008 |
| 1-(3,6-diamino-9H-carbazol-9-yl)-3-(2-(2-(2-fluoroethoxy)ethoxy)ethoxy)propan-1-one | 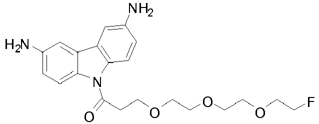 | C₂₁H₂₆FN₃O₄ | 403.45 | |
| N-(2-fluoroethyl)-2-hydroxy-11H-benzo[a]carbazole-3-carboxamide | 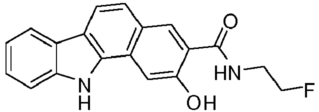 | C₁₉H₁₅FN₂O₂ | 322.33 | |
| 2-(6-chloro-9H-carbazol-2-yl)-N-(2-fluoroethyl)propanamide | 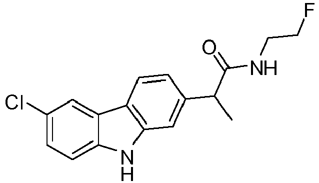 | C₁₇H₁₆ClFN₂O | 318.77 | |
| 2-(6-fluoro-9H-carbazol-2-yl)-N,N-dimethylpropanamide | 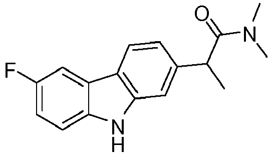 | C₁₇H₁₇FN₂O | 284.33 | |
| 2-methoxy-9H-carbazole | 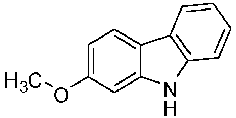 | C₁₃H₁₁NO | 197.23 | |
| 6-iodo-2-methoxy-9H-carbazole | 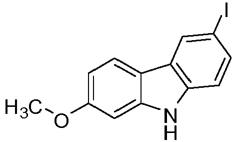 | C₁₃H₁₀INO | 323.13 | |
| 7-(2-fluoroethoxy)-N,N-dimethyl-9H-carbazol-2-amine | 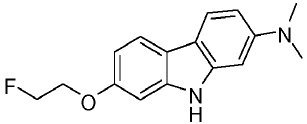 | C₁₆H₁₇FN₂O | 272.32 | |
| tert-butyl 2-(2-(2-(2-fluoroethoxy)ethoxy)ethoxy)-9H -carbazole-9-carboxylate | 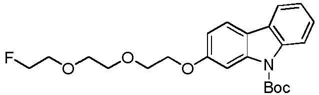 | C₂₃H₂₈FNO₅ | 417.47 | CB-005 |
| 2-(2-(2-(2-fluoroethoxy)ethoxy)ethoxy)-9-methyl-9H-carbazole | 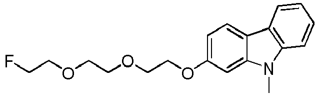 | C₁₉H₂₂FNO₃ | 331.38 | CB-006 |
| 7-(2-(2-(2-fluoroethoxy)ethoxy)ethoxy)-N,N-dimethyl-9H-carbazol-2-amine | 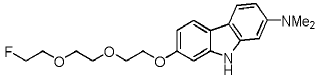 | C₂₀H₂₅FN₂O₃ | 360.42 | CB-007 |
| N-(7-(2-(2-(2-fluoroethoxy)ethoxy)ethoxy)-9H-carbazol-2-yl)acetamide | 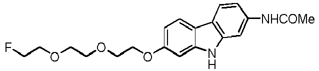 | C₂₀H₂₃FN₂O₄ | 374.41 | CB-009 |
| 7-(2-(2-(2-fluoroethoxy)ethoxy)ethoxy)-9H-pyrido[2,3-b]indole | 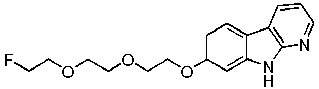 | C₁₇H₁₉FN₂O₃ | 318.34 | CB-028 |
| 2-(2-(2-(2-fluoroethoxy)ethoxy)ethoxy)-9H-carbazole | 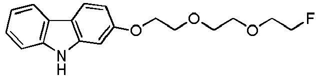 | C₁₈H₂₀FNO₃ | 317.35 | CB-003 |
| 7-(2-(2-(2-fluoroethoxy)ethoxy)ethoxy)-N-methyl-9H-carbazol-2-amine | 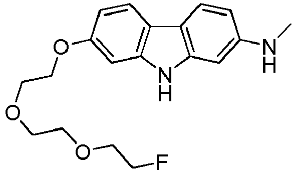 | C₁₉H₂₃FN₂O₃ | 346.40 | CB-004 |
| N-(7-(2-(2-(2-fluoroethoxy)ethoxy)ethoxy)-9H-carbazol-2-yl)formamide | 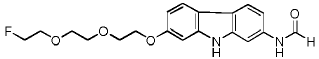 | C₁₉H₂₁FN₂O₄ | 360.38 | CB-010 |
| 6-(2-(2-(2-fluoroethoxy)ethoxy)ethoxy)-9-(methoxymethyl)-N,N-dimethyl-9H-carbazol-3-amine | 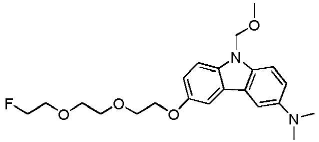 | C₂₂H₂₉FN₂O₄ | 404.48 | CB-011 |
| N-(7-(2-fluoroethoxy)-9H-carbazol-2-yl)formamide | 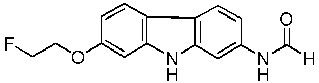 | C₁₅H₁₃FN₂O₂ | 272.27 | CB-012 |
| N-(7-(2-(2-fluoroethoxy)ethoxy)-9H-carbazol-2-yl)formamide | 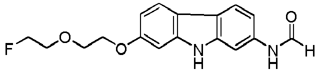 | C₁₇H₁₇FN₂O₃ | 316.33 | CB-024 |
| N-(2-fluoroethyl)-6-methoxy-9H-carbazol-3-amine | 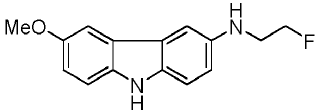 | C₁₅H₁₅FN₂O | 258.29 | CB-013 |
| 7-((4-fluorobutyl)(methyl)amino)-9H-carbazol-2-ol | 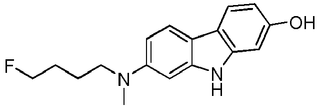 | C₁₇H₁₉FN₂O | 286.34 | CB-014 |
| 7-((2-fluoroethyl)(methyl)amino)-9H-carbazol-2-ol | 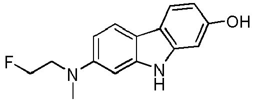 | C₁₅H₁₅FN₂O | 258.29 | CB-015 |
| 7-(2-fluoroethylamino)-9H-carbazol-2-ol | 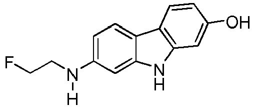 | C₁₄H₁₃FN₂O | 244.26 | CB-016 |
| 7-((2-(2-(2-fluoroethoxy)ethoxy)ethyl)(methyl)amino)-9H-carbazol-2-ol | 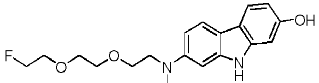 | C₁₉H₂₃FN₂O₃ | 346.40 | CB-019 |
| 7-(2-fluoroethoxy)-N-methyl-9H-carbazol-2-amine | 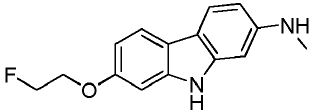 | C₁₅H₁₅FN₂O | 258.29 | CB-020 |
| 7-(2-fluoroethoxy)-9H-carbazol-2-ol | 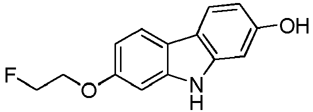 | C₁₄H₁₂FNO₂ | 245.25 | CB-025 |
| 7-(2-(2-(2-fluoroethoxy)ethoxy)ethoxy)-9H-carbazol-2-ol | 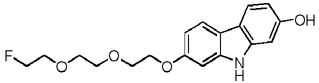 | C₁₈H₂₀FNO₄ | 333.35 | CB-026 |
| N-(4-(7-amino-9H-carbazol-2-yloxy)phenyl)-2-fluoropropanamide | 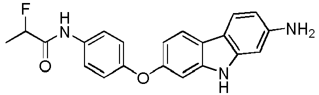 | C₂₁H₁₈FN₃O₂ | 363.38 | CB-027 |
| 1-(2-(2-(2-(2-fluoroethoxy)ethoxy)ethoxy)-9H-carbazol-9-yl)ethanone | 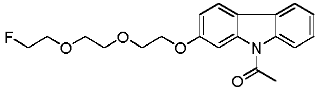 | C₂₀H₂₂FNO₄ | 359.39 | CB-017 |
| (2-(2-(2-(2-fluoroethoxy)ethoxy)ethoxy)-9H-carbazol-9-yl)(phenyl)methanone | 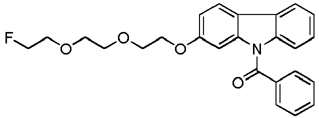 | C₂₅H₂₄FNO₄ | 421.46 | CB-021 |
| 2-fluoro-N-(4-(7-(methylamino)-9H-carbazol-2-yloxy)phenyl)propanamide | 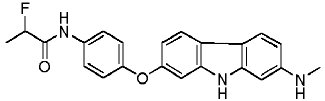 | C₂₂H₂₀FN₃O₂ | 377.41 | CB-029 |
| N-(7-(4-fluorobutoxy)-9H-carbazol-2-yl)formamide | 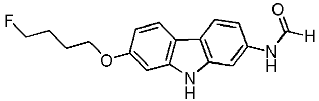 | C₁₇H₁₇FN₂O₂ | 300.33 | CB-030 |
| tert-butyl 2-(2-(2-(2-fluoroethoxy)ethoxy)ethoxy)-9H-pyrido[2,3-b]indol-7-ylcarbamate | 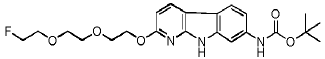 | C₂₂H₂₈FN₃O₅ | 433.47 | CB-031 |
| 2-(2-(2-(2-fluoroethoxy)ethoxy)ethoxy)-9H-pyrido[2,3-b]indol-7-amine | 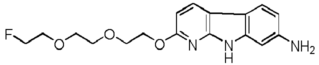 | C₁₇H₂₀FN₃O₃ | 333.36 | CB-032 |
| 7-(benzyloxy)-N-(2-fluoroethyl)-N-methyl-9H-carbazol-2-amine | 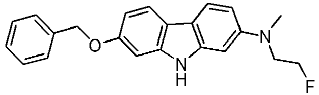 | C₂₂H₂₁FN₂O | 348.41 | CB-033 |
| 2-(2-(2-(2-fluoroethoxy)ethoxy)ethoxy)-N-methyl-9H-pyrido[2,3-b]indol-7-amine | 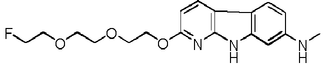 | C₁₈H₂₂FN₃O₃ | 347.38 | CB-034 |
| 6-bromo-9H-carbazol-2-ol | 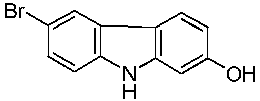 | C₁₂H₈BrNO | 262.10 | |
| 6-(2-fluoroethoxy)-3-hydroxy-2-phenylquinolin -4(1H)-one | 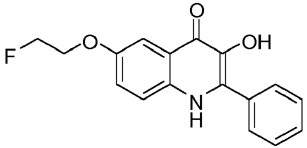 | C₁₇H₁₄FNO₃ | 299.30 | |

**The following compounds in Table 3 are included for reference purposes:**

| **ID** | **T807** | **T805** | **T703** | **T794** |
|---|---|---|---|---|
| | 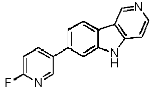 | 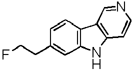 | 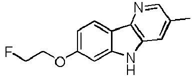 | 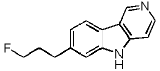 |
| **MW** | **360.3** | **214.2** | **244.3** | **228.3** |
| **CLogP** | **3.2** | **2.8** | **3.6** | **3.4** |
| **KD (tau)** | **15 nM** | | **32nM** | **30 nM** |
| **selectivity (tau/Ab)** | **29x** | **14x** | **16x** | **17x** |
| **GM Intensity (comp. with W372)** | **28%** | **8%** | **6%** | **14%** |
| **background (normal brain 32566) PSL/mm2** | **61** | **49** | **15** | **97** |
| **Tx/tau/amyl oid correlation** | **yes** | | | **yes** |
| **Brain uptake** | **yes (3 rats, 3 mice)** | **yes (2 rats, 2 mice)** | **Yes** | **yes (4 rats, 4 mice)** |
| **in vivo metabolism** | **15, 30 min** | **15 min** | **Yes** | **5, 15,30 min** |
| **in vivo metabolism : one day** | | | | |
| **human hepatocytes** | **somewhat stable (less polar metabolite)** | | | **stable** |
| **PK** | **yes** | **yes** | **yes** | **yes** |
| **45 brain panel** | **done** | | | **done** |
| **double staining** | | | | **yes** |
| **AchE activity (IC50)** | **6 uM** | **<50% at 10 uM** | | **5 uM** |
| **MAO inhibition** | 0% (1 uM) MAO-A 0% (1 uM) MAO-B | 0% (1 uM) MAO-A 0% (1 uM) MAO-B | 16% (1µM) MAO-A 0% (1µM) MAO-B | 19% (1 uM) MAO-A 0% (1 uM) MAO-B |
| **CNS** | | | **NET, σ1** | **1 uM** |
| **selectivity panel** | | | | Norepinephrine transp.(49%) Dopamine transp.(34%) Glutamate, NMDA (44%) Monoamine transp.(41%) Serotonin 5-HT2c (33%) |
| **MDS SDL/quote#** | SDL-20, #22673 | | | SDL-18, #22639-1 (5 targets at 2 more concentrations) |

**The following compounds in Table 4 are included for reference purposes:**

| **ID** | **Stucture** | **MW** | **CLog P** | **compound dbl staining (100 uM)** | **KD (tau)** | **KD (amyloid)** |
|---|---|---|---|---|---|---|
| T734 | 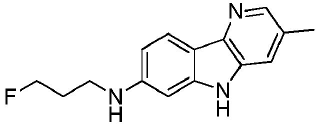 | 257.31 | 3.5 | blue/green . Tau+. Other- | | |
| T733 | 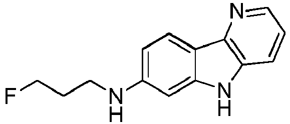 | 243.3 | 3.0 | blue/green . Tau-. Other- | | |
| T728 | 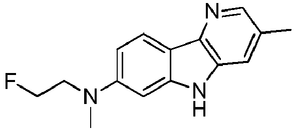 | 257.3 | 3.8 | green/blue . Tau+. Other- | | |
| T726 | 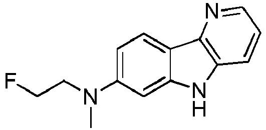 | 243.3 | 3.3 | green/blue . Tau+++. Other+ | | |
| T687 | 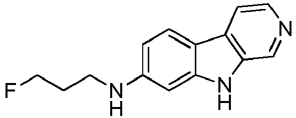 | 243.3 | 2.8 | blue. Tau++++. Ab++ | | |
| T686 | 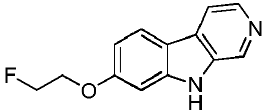 | 230.2 | 2.9 | blue. Tau+. Other+ | | |
| T660 | 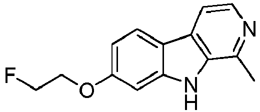 | 244.3 | 3.4 | Tau-. Ab+ | | |

It will be understood that the halogen of these carbazole-based compounds, for example, F, may be radioactive or it may be "cold." In particular, it may be ¹⁸F. Other suitable radioactive atoms may include ¹¹C, ¹³N, ¹⁵O, ¹⁸F, ⁶¹Cu, ⁶²Cu, ⁶⁴Cu, ⁶⁷Cu, ⁶⁸Ga, ¹²⁴I, ¹²⁵I, ¹³¹I, ⁹⁹Tc, ⁷⁵Br, ¹⁵³Gd and ³²P.
For example, radiolabeled compounds may include:

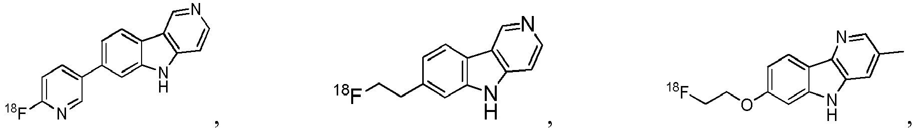

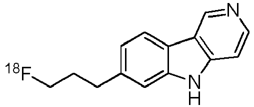

Precursors of the compounds of the present invention may be: 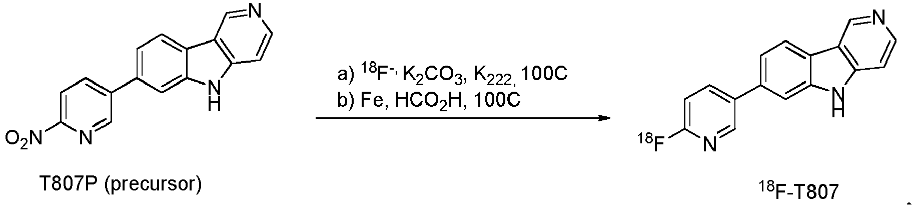 Other precursors disclosed herein include:

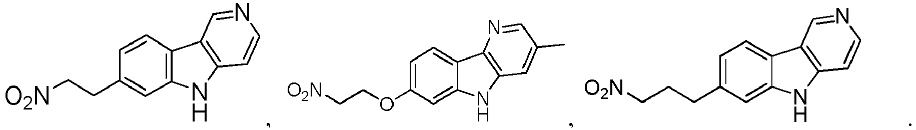

Compounds of the present invention may also be: 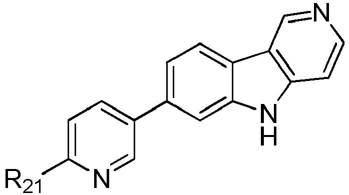 wherein R₂₁ is halo or a radionuclide.

When injected intravenously into mice, the Carbazole based compounds; in particular T807, T805 and T794 have shown excellent brain uptake. These compounds also display high binding affinity to tau fibrils. Autoradiography using the present compounds demonstrated labeling of NFTs in AD brain sections. Fluorescence assay data shows the binding ability of these agents to tau aggregates and Aβ fibrils. In neuropathological staining, compounds of the present invention stained amyloid plaques and/or tau aggregates.

In another embodiment, the present invention relates to compounds and compositions which comprise the formulae as disclosed herein, wherein the compound is an amyloid and/ or tau protein binding compound. An amyloid and/or tau protein binding compound of the invention may be administered to a patient in amounts suitable for in vivo imaging of amyloid deposits and/or NTFs, and distinguish between neurological tissue with amyloid deposits and/or NTfs and normal neurological tissue.

Aβ compounds are typically evaluated in a competitive binding assay using synthetic Aβ1-42 fibrils (IC₅₀s). The situation is more complicated for tau, because there are 6 isoforms of tau potentially present in AD brains as products of alternate splicing of a single tau gene. Most reports in the literature rely therefore on only one recombinant isoform, Tau-441. To add more complexity, the various tau isoforms are hyperphosphorylated in vivo, something that is difficult to mimic in vitro. Furthermore, structural information on these tau fibrils is lacking, making an interpretation of binding of compounds difficult.

Native forms of tau (various isoforms, hyperphosphorylated) and amyloid aggregates are present in brain sections and therefore preferred for compound testing. Using the self-fluorescence of a test compound can give an indication of whether the compound binds to tau tangles/PHFs and/or amyloid plaques. This is further confirmed by immunostaining with Aβ and tau antibodies and overlaying the images. The drawback is that the fluorescent signals cannot be used for quantitation as some compounds might exhibit a stronge fluorescent signal than others and the coexistence of Aβ plaques and tau tangles in AD brains. However, it is possible to "rate" the signal strength qualitatively and distinguish compounds that show binding to these aggregates.

Furthermore, the selectivity can be evaluated in brains containing only Aβ plaques/no tau aggregates, Aβ plaques/and tau aggregates, and control brains. Unfortunately, there are no AD brains with only tau and no Aβ present. By testing radiolabeled tracers in these brain sections, one can more quantitative evaluate the relative binding strength (signal strength) and selectivity of various test compounds as they all contain the same radioactive tracer. For examples, if a test tracer binds only to tau, and not amyloid, it should show no signal in the Aβ plaques only brain sections. If a compound binds only to amyloid, it should show uptake in both types of brains. The difficulty of identifying and further quantifying selective compounds lies in the relative abundance of amyloid vs. tau, which is difficult to measure.

Amyloid and/or tau protein probes of the invention may be used to detect and quantitate amyloid deposits and/or NTFs in diseases including, but not limited to Mediterranean fever, MuckleWells syndrome, idiopathetic myeloma, amyloid polyneuropathy, amyloid cardiomyopathy, systemic senile myloidosis, amyloid polyneuropathy, hereditary cerebral hemorrhage with amyloidosis, Down's syndrome, Scrapie, Creutzfeldt-Jacob disease, Kuru, Gerstamnn-Straussler-Scheinker syndrome, medullary carcinoma of the thyroid, Isolated atrial amyloid, β₂-microglobulin amyloid in dialysis patients, inclusionbody myositis, β₂-amyloiddeposits in muscle wasting disease, chronic traumatic encephalopathy (CTE), and Islets of Langerhans diabetes Type II insulinoma.

In other embodiments of the invention, the labeled compound is introduced into a patient in a detectable quantity and after sufficient time has passed for the compound to become associated with amyloid deposits and/or tau proteins, the labeled compound is detected noninvasively. Herein disclosed isa labeled compound of the Formulas disclosed herein is introduced into a patient, sufficient time is allowed for the compound to become associated with amyloid deposits, and then a sample of tissue from the patient is removed and the labeled compound in the tissue is detected apart from the patient. Herein disclosed is a method by which tissue sample is removed from a patient and a labeled compound of Formula 8, for example, is introduced into the tissue sample. After a sufficient amount of time for the compound to become bound to amyloid deposits and/or tau proteins, the compound is detected.

### Synthesis of ligands and their labeling precursors:

### Halogenation and Radiohalogenation:

As disclosed herein, for a number of different AD ligands, such as flavones, coumarins, carbazoles, quinolinones, chromenones, trisubstituted imidazoles and their derivatives as disclosed herein, the radiolabeled atom, such as a halogen atom, for example, may be readily introduced into the ligand using a number of different methods well known in the art. Accordingly, the radiolabeled compounds of the Formulas 7-8 may be prepared using standard methods known in the art for preparing such radiolabeled compounds having a particular substituent, wherein the compound may be incorporated with a particular radionuclide selected from the group consisting of ¹¹C, ¹³N, ¹⁵O, ¹⁸F, ¹²³I, ¹²⁴I, ¹²⁵I, ¹³¹I and ⁷⁷Br.

In one particular example, the halogen may be introduced by a method using a tin for halogen exchange process. For example, a non-radioactive halogen such as iodine, may be replaced by an organo tin compound via a metal, such as a palladium composition, to form the radio labeling tin precursor, as represented below. This precursor is then subjected to radioactive halogenation via displacement with Na¹²⁵I source, for example, to afford the radioactive ligand.

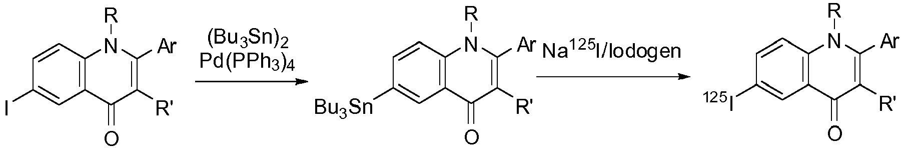

Alternatively, the radio labeled halogen may be readily introduced via direct halogenation. For example, for a ligand comprising an aromatic ring as part of the scaffold, or an aromatic substituent of a ligand, the aromatic ring may be directly iodinated using well-established radioiodination procedure. One such example is represented below using a carbazole ligand.

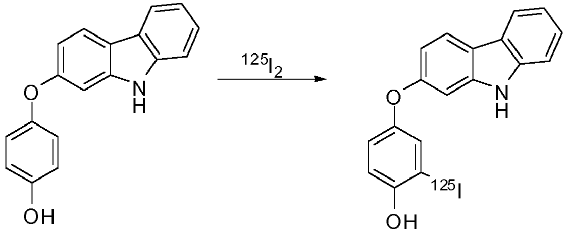

For ¹¹C-labeled compounds, the labeled compound may be prepared by the alkylation or methylation of a hydroxyl group, such as with [¹¹C]CH₃I to provide the corresponding C-11 labeled methoxy derivative. For example, such a process is represented by the reaction of the flavone derivative shown below.

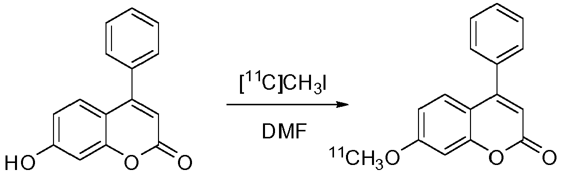

Other methods of preparing radiolabeled ligands are well known in the art. Example of such methods are disclosed in, for example: 1) Jewett, D.M. (1992) A Simple Synthesis of [11C]Methyl Triflate Appl. Radiat. Isot. 43, 1383-1385; 2) Crouzel, C. Langstrom, B., Pike, V.W., and Coenen, H.H. (1987) Recommendations for a practical production of [11C]methyl iodide Appl. Radiat. Isot. Int. J. Appl. Instrum. Part A 38, 601-603; Dannals, R.F., Ravert, H.T.; 3) Wilson, A.A. (1990) Radiochemistry of Tracers for Neurotransmitter Receptor Studies. In: Quantitative Imaging: Neuroreceptors, Neurotransmitters, and Enzymes. (Edited by Frost, J.J. Wagner Jr., H.N. pp. 19-35, Raven Press, New York; 4) Jewett, D.M., Manger, T.J., and Watkins, G.L. (1991) Captive Solvent Methods for Fast Simple Carbon-11 Radioalkylations. In: New Trends in Radiopharmaceutical Synthesis, Quality Assurance and Regulatory Control (Edited by Emran, A.M.) pp. 387-391. Plenum Press, New York; 5) Marazano, C., Maziere, M., Berger, G., and Comar, D. (1977) Synthesis of methyl iodide-11C and formaldehyde-11C Appl. Radiat. Isot. 28, 49-52; 6) Watkins, G., Jewett, D., Mulholland, G., Kitbourn, M., and Toorongian, S. (1988) A Captive Solvent Method for Rapid N-[11C]Methylation of Secondary Amides: Application to the Benzodiazepine, 4'-Chlorodiazepam (RO5-4864) Appl. Radiat. Isot. 39, 441-444; and 7) Wilson, A. A., DaSilva, J.N., and Houle, S. (1996) In vivo evaluation of [11C] and [15F]-labelled cocaine analogues as potential dopamine transporter ligands for positron emission tomography Nucl. Med. Biol. 23, 141-146.

### Assays of Carbazole Derivatives:

From the Biacore assay, two carbazole derivatives displayed promising binding affinities to oligomers/polymers and fibrils (Table 4). The beta-carboline Harmol, a member of the harmala alkaloids, is the urinary metabolite of harmine. The harmala alkaloids are MAO inhibitors and are commonly found in Syrian rue, *Peganum harmala*, and the South American vine *Banisteriopsis caapi,* both of which are purported to possess strong hallucinogenic effects. The beta-carbolenes have a varied effect on the central nervous system including binding to the 5-HT₂, 5-HT₁ₐ, glutamate NMDA and imidazoline receptors; inhibiting MAO-A enzyme and interfering with dopaminergic transmission. And while beta-carbo lines are thought to be cytotoxic, they also maintain neuroprotective properties supposedly offering neuroprotection against dopamine and glutamate and, additionally, by scavenging reactive oxygen species. A recent report demonstrated that beta-carboline alkyloids induce a facilitation of short and long term memory in object recognition tasks in mice, although the means by which the alkyloids are exerting their effect is unclear. Moura, D.J., et al., Effects of b-carboline alkaloids in the object recognition task in mice. Life Sciences, 2006, 79: p. 2099-2104.

The second active carbazole discovered in the assay is 2-hydroxycarbazole. 2-Hydroxycarbazole has been recently shown to release Ca²⁺ ion from skeletal and cardiac muscle through a distinct pharmacological pathway. The generic carbazole scaffold exists in several therapeutics including the non-steroidal anti-inflammatory carprofen, carazolol (a beta-blocker) and YM-53601 (a squalene synthase inhibitor). Recent work has shown that carbazole derivatives can act as γ-secretase modulators. [Narlawar, R., et al., N-Substituted carbazolyloxyacetic acids modulate Alzheimer associated g-secretas. Bioorganic & Medicinal Chemistry Letters, 2007, 17: p. 176-182] In another AD related project, Howlett discovered highly elaborated carbazoles, such as carvedilol, inhibit fibril formation, albeit the binding affinities to the fibrils were not determined. [Howlett, D.R., et al., Common Structural Features Determine the Effectiveness of Carvedilol, Daunomycin and Rotiletracycline as Inhibitors of Alzheimer b-Amyloid Fibril Formation. Biochemical Journal, 1999, 343: p. 419-423] Interestingly, an article intending to determine the practicality of using carbazoles as fibril inhibitors based on cell permeability suggests that carbazoles are unlikely to cross the blood brain barrier, as they are PGP substrates, precluding their use as therapeutics for fibril inhibition. [Saengkhae, C., et al., Ability of Carbazole Salts, Inhibitors of Alzheimer b-Amyloid Fibril Formation, to Cross Cellular Membranes. European Journal of Pharmacology, 2007, 559: p. 124-131]

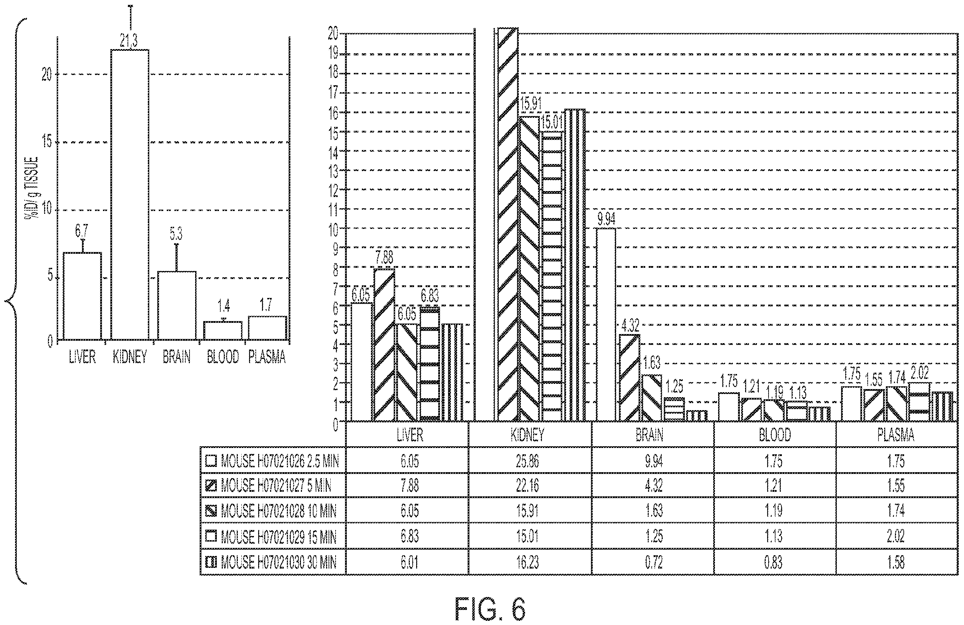

By using an appropriate imaging modality, a tracer's biodistribution pattern becomes instantly visible and accessible. For example, by using ¹⁸F-labeled tracers one can easily quantify a tracer's uptake into, and washout from, the brain using positron emission tomography (PET). Tracers with high uptake and slow washout in normal brains generate low signal to noise ratios. Tracers with high uptake and fast washout in normal brains have high signal to noise rations and are considered ideal. ¹⁸F-labeled carbazoles possess ideal brain imaging properties. For example, an ¹⁸F-labeled carbazole was prepared and administered to a normal, white Sprague-Dawley rat (Figure 6 of USSN 12/372,717). Within minutes, the tracer entered into the brain and washed out over several minutes.

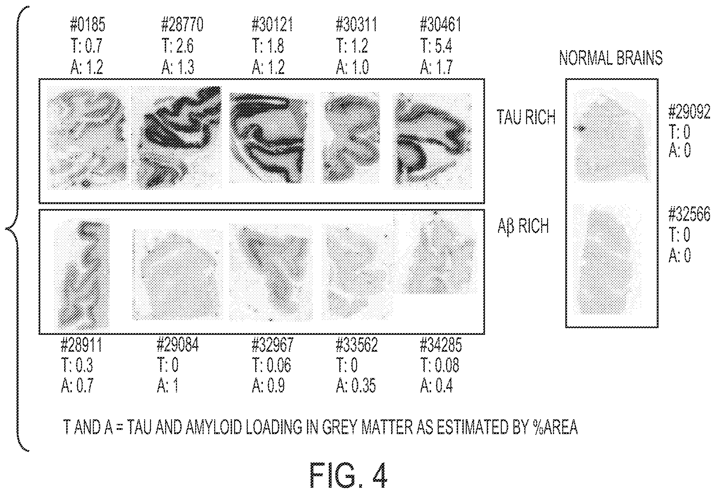

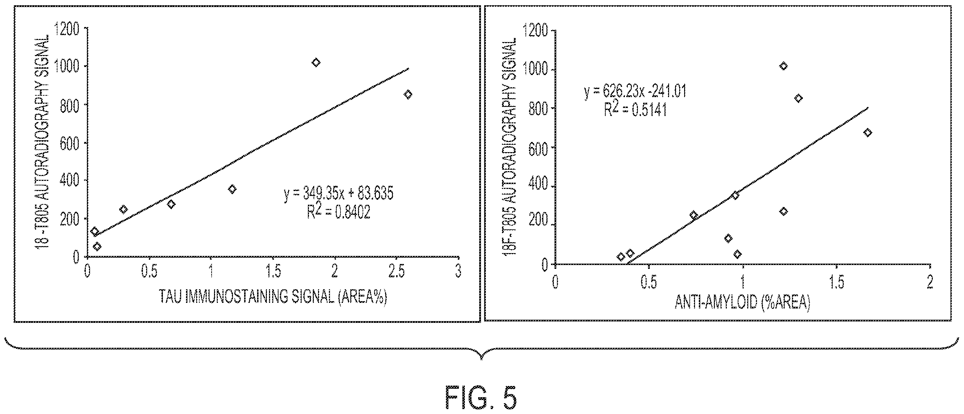

The non-radioactive carbazole also successfully competes off both Thioflavin T and FDDNP in brain tissue sections suggesting that the tracer binds to similar binding sites (Figures 4 and 5 of USSN 12/372,717).

**Table 4: Carbazole-based hits from the Biacore assay. A "+" sign represents a hit and the increase in "+" signs relates to increasing binding affinity. A "-" sign represents no binding.**

| | Binding to oligomers/polymers (Aβ1-42) | Binding to fibrils (Aβ1-42) |
|---|---|---|
| #54: Harmol | | |
| 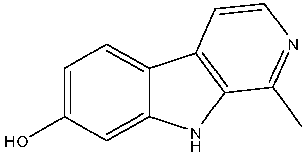 | ++ | + |
| #55: 2-Hydroxycarbazole | | |
| 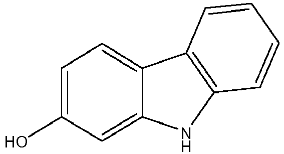 | +++ | + |
| #73: 7,8-Dihydroxy-4-phenylcoumarin | | |

A list of examples of carbazole-based imaging agents are shown in Table 5. Many of the compounds are either ¹⁸F- or ¹¹C-labeled.

**Table 5: Examples of carbazole-based imaging agents, which are included for reference purposes. Any of these may include a halogen and/or a radionuclide or may be "cold." The halogen may be replaced with a radionuclide such as ¹⁸F.**

| **Compound Name** | **Structure** | **Formula** | **Mol.Weight** |
|---|---|---|---|
| 2-(2-fluoroethoxy)-9H-carbazole | 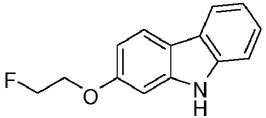 | C₁₄H₁₂FNO | 229.25 |
| 9-(2-fluoroethyl)-9H-carbazol-2-ol | 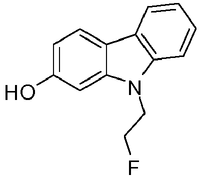 | C₁₄H₁₂FNO | 229.25 |
| N-(2-fluoroethyl)-7-(2-(2-(2-methoxyethoxy)ethoxy)ethoxy)-9H-carbazol-3-amine | 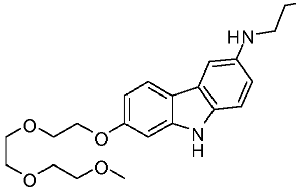 | C₂₁H₂₇FN₂O₄ | 390.45 |
| 7-(2-fluoroethoxy)-N,N-dimethyl-9H-carbazol-2-amine | 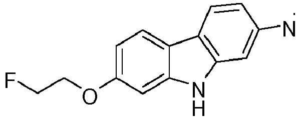 | C₁₆H₁₇FN₂O | 272.32 |
| 7-(2-(2-(2-fluoroethoxy)ethoxy)ethoxy)-N-methyl-9H-carbazol-3-amine | 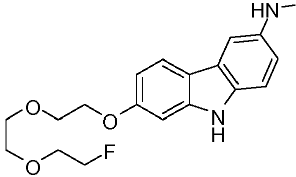 | C₁₉H₂₃FN₂O ₃ | 346.40 |
| 1-(3,6-diamino-9H-carbazol-9-yl)-3-(2-(2-(2-fluoroethoxy)ethoxy)ethoxy)propan-1-one | 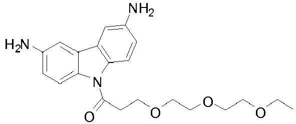 | C₂₁H₂₆FN₃O ₄ | 403.45 |
| N-(2-fluoroethyl)-2-hydroxy-11H-benzo[a]carbazole-3-carboxamide | 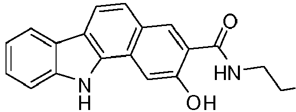 | C₁₉H₁₅FN₂O ₂ | 322.33 |
| 2-(6-chloro-9H-carbazol-2-yl)-N-(2-fluoroethyl)propanamide | 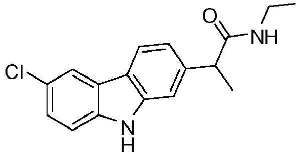 | C₁₇H₁₆ClFN₂ O | 318.77 |
| 2-(6-fluoro-9H-carbazol-2-yl)-N,N-dimethylpropanamide | 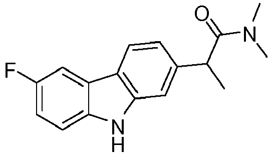 | C₁₇H₁₇FN₂O | 284.33 |
| 2-methoxy-9H-carbazole | 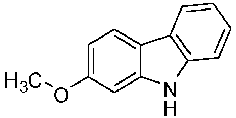 | C₁₃H₁₁NO | 197.23 |
| 6-iodo-2-methoxy-9H-carbazole | 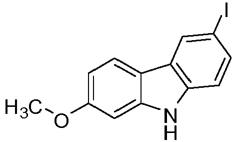 | C₁₃H₁₀INO | 323.13 |

### Detailed Biacore assay protocol:

**β-Amyloid (Aβ42) soluble aggregates (oligomers/soluble polymers).** Biotin-LC-Aβ42 was mixed with Aβ42 at a ratio of 3:2. After dissolving in 1% NH₄OH and dH₂O, the mixture (40 uM concentration) was incubated in IX PBS (pH 7.4) buffer at RT for 6-hours to form oligomers/soluble polymers. The free monomer of Aβ42 in the sample was removed using a Microcon centrifugal filter tube with a 10 KDa of MW cutoff. The Biotin-LC-Aβ42 oligomers/polymers were immobilized onto SA chip by streptavidin-biotin capture.

**β-Amyloid (Aβ42) insoluble aggregates (fibrils).** Fibrils were prepared according to methods published previously (Agdeppa ED et al. 2001). Briefly, 0.5 mg of Aβ42 (Biotin-LC-Aβ42:Aβ42 = 1:1) was dissolved in 1 ml of PBS, pH 7.4, and mixed with a magnetic stir bar for 3 d at 37 °C, resulting in a visibly cloudy solution. The fibril pellet was collected by centrifugation. The Biotin-LC-Aβ42 fibrils were immobilized onto SA chip by streptavidin-biotin capture.

**Screening of amyloid binding compounds with Biacore (Surface Plasmon Resonance Analysis).** Aβ42 oligomers/soluble polymers or fibrils were immobilized on Flow Cell 2 (Fc2) or Flow Cell 3 (Fc3) of the Sensor Chip, with Fc1 serving as the control. Screening compounds at 10 uM concentration was flown through Fc1, Fc2, and Fc3 for 2 minutes at a flow rate of 30 ul/minute. The Flow Cells were then washed with running buffer (IX PBS) for 2 minute, and regenerated with 50 mM of NaOH for 30 seconds. The real time interaction between the screening compound and the amyloid aggregates immobilized on the chip surface was recorded in the sensorgram.

**Immunostaining of brain sections with Thioflavin T.** Brain samples from donors with Alzheimer disease were paraffin wax infiltrated after fixation. Paraffin blocks with embedded brain samples were mounted onto microtome and sectioned. Sections were then deparaffinized and hydrated, followed by incubation with or without AD-CB-001S-WZ01013. Staining was carried out with 1 uM Thioflavin T. Images were obtained with a fluorescence microscope (Figure 4 of USSN 12/372,717).

**Immunostaining of brain sections with FDDNP.** Brain samples from donors with Alzheimer disease were paraffin wax infiltrated after fixation. Paraffin blocks with embedded brain samples were mounted onto microtome and sectioned. Sections were then deparaffinized and hydrated, followed by incubation with or without AD-CB-001S-WZ01013. Staining was carried out with 1 uM FDDNP. Images were obtained with a fluorescence microscope (Figure 5 of USSN 12/372,717).

### Imaging Results of AD-CB-001

A white Sprague-Dawley rat was injected via tail vein with ∼850 uCi AD-CB-001, formulated in 10% EtOH:water. A dynamic scan was conducted for 30 min on a R4 microPET scanner. The data was reconstructed using 1 min framing. Within minutes, the tracer entered the rat brain and quickly washed out (Figure 6 of USSN 12/372,717).

### General Procedure for Carbazole N-Boc Protection:

To a round bottomed flask equipped with a magnetic stir bar, rubber septum, and argon inlet containing THF (40 vol) was placed carbazole (1.0 equiv). To this solution was added NaH (60% dispersion in oil, 3 equiv) at 0 °C and the reaction was allowed to stir at 0 °C for 30 min. To this reaction was added (Boc)₂O (1.2 equiv) at 0 °C and the reaction was allowed to stir for 1 h. After the reaction was complete by LCMS, poured into water (25 vol) and extracted into EtOAc (3 x 20 vol). The combined organic extracts were washed with water (2 x 25 vol), dried (Na₂SO₄) and concentrated *in vacuo.* The residue was purified over silica gel using Hexanes:EtOAc as an eluent to afford the final product.

### General Procedure for Carbazole N-methylation:

To a round bottomed flask equipped with a magnetic stir bar, rubber septum, and argon inlet containing THF (50 vol) was placed carbazole (1.0 equiv). To this solution was added NaH (60% dispersion in oil, 3 equiv) at 0 °C and the reaction was allowed to stir at 0 °C for 30 min. To this reaction was added MeOTf (1.0 equiv) at 0 °C and the reaction was allowed to stir for 1 h. After the reaction was complete by LCMS, poured into water (25 vol) and extracted into EtOAc (3 x 20 vol). The combined organic extracts were washed with water (2 x 25 vol), dried (Na₂SO₄) and concentrated *in vacuo.* The residue was purified over silica gel using Hexanes:EtOAc as an eluent to afford the final product.

### General experimental procedure for phenolic alkylation:

To a round bottomed flask equipped with a magnetic stir bar containing DMF (20 vol) was placed **phenol** (1 equiv). To this solution was added **alkylating agent** (1.0 equiv), Cs₂CO₃ (1.2 equiv) and the reaction was allowed to stir at 60 °C for 16 h. The reaction was then poured into water (25 vol) and extracted into EtOAc (3 x 20 vol). The combined organic extracts were washed with water (2 x 25 vol), dried (Na₂SO₄) and concentrated *in vacuo.* The residue was purified over silica gel using Hexanes:EtOAc as an eluent to afford the final product.

### General experimental procedure for Suzuki coupling reaction:

To a round bottomed flask equipped with a magnetic stir bar rubber septum, and argon inlet containing toluene:H₂O (1:1, 40 vol) was placed **chloro compound** (1 equiv). To this solution was added **boronic acid** (1.5 equiv), Pd(PPh₃)₄ (0.02 equiv), K₂CO₃ and the reaction was allowed to stir at 110 °C for 16 h. The reaction was then poured into water (25 vol) and extracted into EtOAc (3 x 20 vol). The combined organic extracts were washed with water (2 x 25 vol), dried (Na₂SO₄) and concentrated *in vacuo.* The residue was purified over silica gel using Hexanes:EtOAc as an eluent to afford the final product.

### General experimental procedure for Carbazole formation using P(OEt)₃:

To a round bottomed flask equipped with a magnetic stir bar containing P(OEt)₃ (25 vol) was placed **biaryl** (1 equiv). The reaction was allowed to stir at 150 °C for 16 h. After the reaction was complete, P(OEt)₃ was removed *in vacuo.* The residue was purified over silica gel using Hexanes:EtOAc as the eluent to afford the final compound.

### Ex vivo competition assay using Amyloid (AD patient's brain slice) Autoradiography Staining

The carbazole series of AD imaging agents display surprisingly good qualities when compared to previously established results performed by others. Data from prior art suggests that compounds with higher LogP values have higher amyloid affinities, yet these same compounds can also suffer from high non-specific binding, i.e poor brain washout (J. Molecular Neuroscience 2003,20, 255-260). For the disclosed studies in this application, cLogP values were used in place of LogP values.

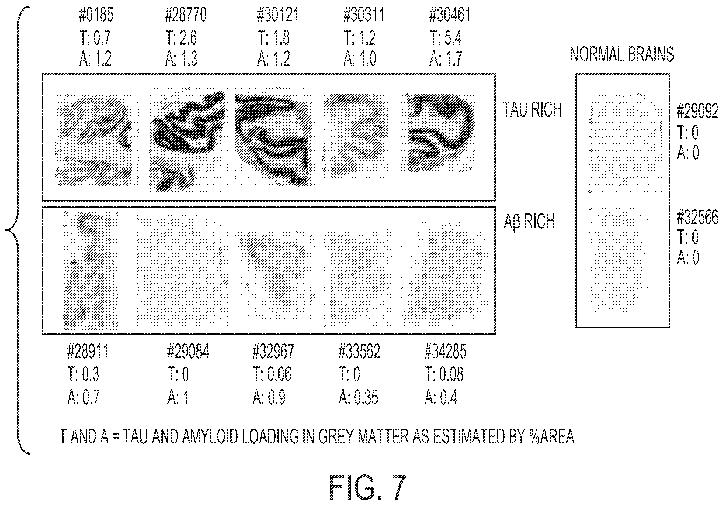

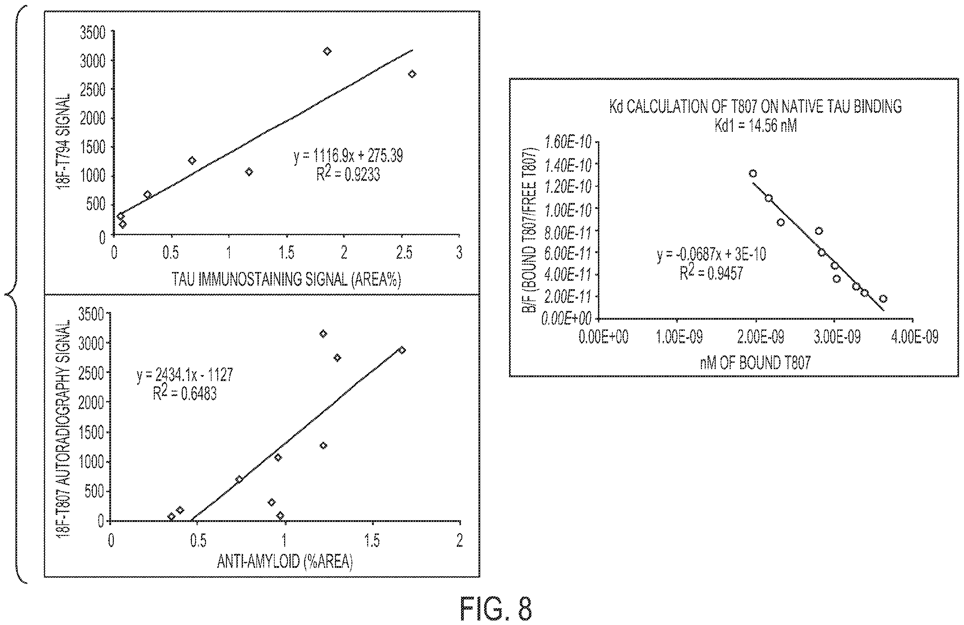

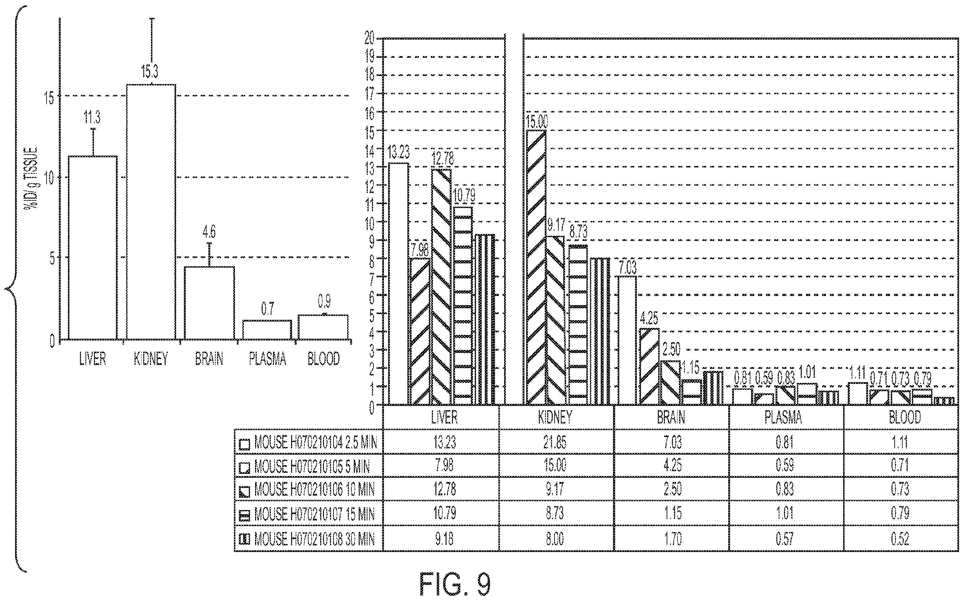

A study was conducted to examine the grey to white matter binding ratios for 4 different tracers: CB-001, CB-003, FDDNP and F-PiB (Figure 7 and Figure 8 of USSN 12/372,717). A known carbazole containing imaging agent, 18F-fluorocarazolol, was not examined in this study because of its relatively low cLogP value (2.77) compared to FDDNP and PiB, and its competing specific uptake into the beta-adrenoceptors. In addition, there is no prior art data suggesting that 18F-fluorocarazolol binds to AD plaques. After the human brain slices from AD patients were incubated with a given tracer for 30 min, the slices were washed with various EtOH:water solutions in an attempt to optimize the grey to white matter ratios (Figure 9 of USSN 12/372,717). The results were surprising and unexpected in view of previous work performed by other researchers. CB-001 has a slightly higher cLogP than FDDNP (3.8 vs 3.4) and would be expected to have poorer washout than FDDNP based on these values. However, despite the difference in cLogP values, CB-001 has a lower non-specific binding propensity and displays a much better grey to white matter ratio compared to FDDNP (see section above, "original wash"). More specifically, the white matter binding of FDDNP is several shades darker than CB-001's white matter binding, indicating low non-specific binding of CB-001. In contrast, F-PiB, which has a cLogP value of 3.99, also displays reasonable, binding ratios similar to CB-001, albeit displaying a very weak overall signal. The washing data suggests that the carbazoles are a viable and novel target for imaging AD-related targets due to their unique binding and washout properties.

To expand on these results, CB-003, a tracer with a cLogP value similar to FDDNP, was prepared and tested. Using washing conditions that were far milder than the harsh washing conditions (Figure 9 of USSN 12/372,717), CB-003 displayed excellent grey to white matter binding ratios that are far superior to the results taken from FDDNP, PiB and CB-001. These favorable and unique results suggest that CB-003 would have a more favorable brain washout in living systems, leading to more specific uptake and lowered non-specific binding, leading to a clear advantage over FDDNP and PiB imaging.

### Summary of Washing Results:

| **Name** | **Structure** | **cLogP** | **Grey/white matter binding ratio using harsh FDDNP wash conditions*** | **Grey/white matter binding ratio using mild wash conditions**** |
|---|---|---|---|---|
| CB-001 | 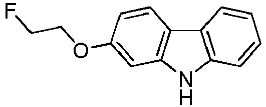 | 3.789 | **Excellent** | Poor |
| CB-003 | 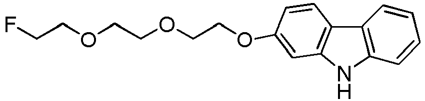 | 3.4032 | N/A | **Excellent** |

| | | | | |
|---|---|---|---|---|
| * published FDDNP wash conditions: 30 min incubation of CB-1 or CB-3 tracer, PBS wash (5 min), 70% EtOH:water (1 min), 90% EtOH:water (1 min), 70% EtOH:water (1 min), PBS (5 min). The brain slices were 20 um thick. ** mild wash conditions: 30 min incubation of CB-1 or CB-3 tracer, PBS wash (5 min), 30% EtOH:water (2 min), 40% EtOH:water (2 min), 20% EtOH:water (2 min), PBS (5 min). The brain slices were 20 um thick. | | | | |

The results demonstrate that 1) PiB blocks [18F]-CB001 staining with increasing concentrations, suggesting the two compounds to compete for the same amyloid binding pockets; 2) PiB appears to block tracer binding with the same strength as cold CB001, suggesting both to have similar binding affinities; 3) FDDNP is much less capable of blocking [18F]-CB001 staining, due to its lower amyloid binding affinity.

This data suggests the following order of (non-specific) white matter binding: FDDNP > CB001 > [18F]-PiB > CB003

### IC50 Determination with [18F]-PiB by ex vivo competition assay using autoradiography staining

| **Compound Code** | **IC50** | | | | | | | | | | | | | **Average IC50** | **SD** | **SD %** |
|---|---|---|---|---|---|---|---|---|---|---|---|---|---|---|---|---|
| | **1** | **2** | **3** | **4** | **5** | **6** | **7** | **8** | **9** | **10** | **11** | **12** | **13** | | | |
| **F-PiB** | | | | | | 43 | | 43 | 40 | 50 | 55 | 41 | | **45** | 6 | 13 |
| **PiB** | 80 | 40 | 40 | 48 | 60 | 43 | 50 | | | | | | 280 | **52** | 14 | 28 |
| **CB7** | 260 | | 170 | | 200 | 290 | | | | | | | 300 | **244** | 57 | 23 |
| **CB4** | 260 | | 350 | | 300 | 300 | | | | | | | 400 | **322** | 54 | 17 |
| **CB12** | | | | 610 | 300 | 450 | 390 | | | | | | | **438** | 130 | 30 |
| **CB24** | | | | | | | 540 | | | | | | | **540** | | |
| **CB1** | 1000 | 480 | | | | | | | | | | | | **740** | 368 | 50 |
| **CB10** | | | | 900 | | | | | | | | | | **900** | | |
| **CB3** | 1100 | | | | | 900 | | | | | | | 920 | **973** | 110 | 11 |

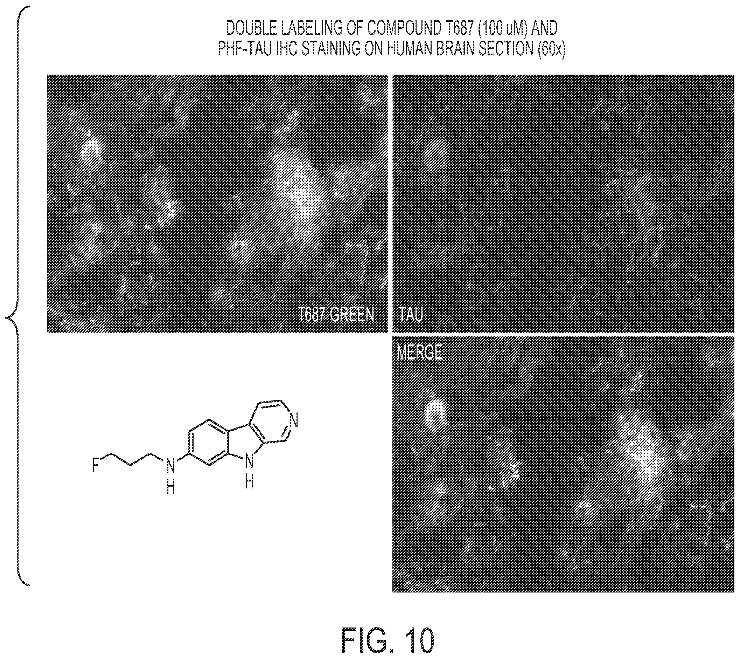

To further demonstrate the efficiency of employing these CB-related tracers as AD imaging agents, CB-003 was used to clearly differentiate between a healthy brain and an AD brain (Figure 10 of USSN 12/372,717). More specifically, by using the mild wash protocol, the amyloid deposits were clearly visible in the grey matter with little white matter uptake. The results were corroborated by both antibody IHC and thioflaving T amyloid staining, confirming the specificity of uptake. These surprising results demonstrate that this tracer possess the unique quality of rapid washout from white matter and significant high uptake in grey matter that is specific for AD plaques.

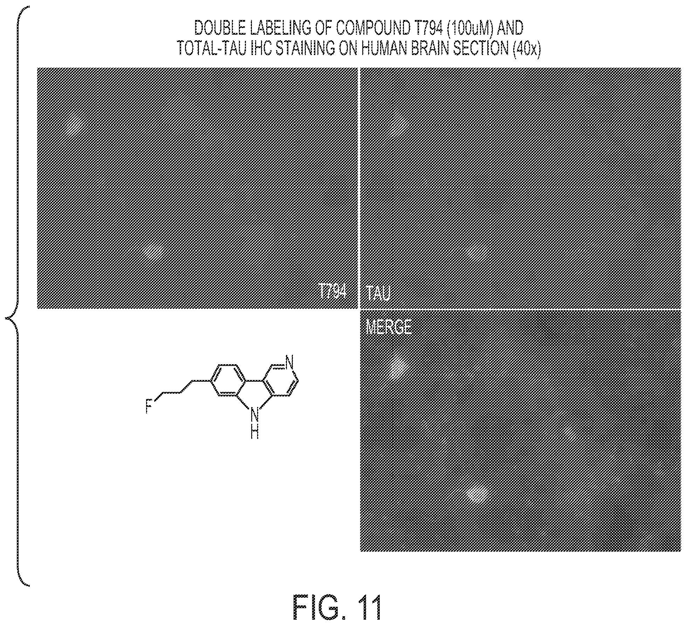

The carbazoles compete directly against 18F-PiB for the same binding sites in human AD brains (Figure 11 of USSN 12/372,717). This surprising result could not have been predicted given their dissimilar structures and CB-003's lack of a phenolic OH and terminal NH-Me group, which are deemed essential for binding to AD plaques. Despite CB-003 lacking both of these functional groups, it still competes with 18F-PiB for binding sites in human AD brains. Because of the simplicity of its structure, the labeling yields of CB-001 and CB-003 are exceptionally high and better than the labeling yields of 18F-PiB.

### Surface Plasmon Resonance (SPR) Assay

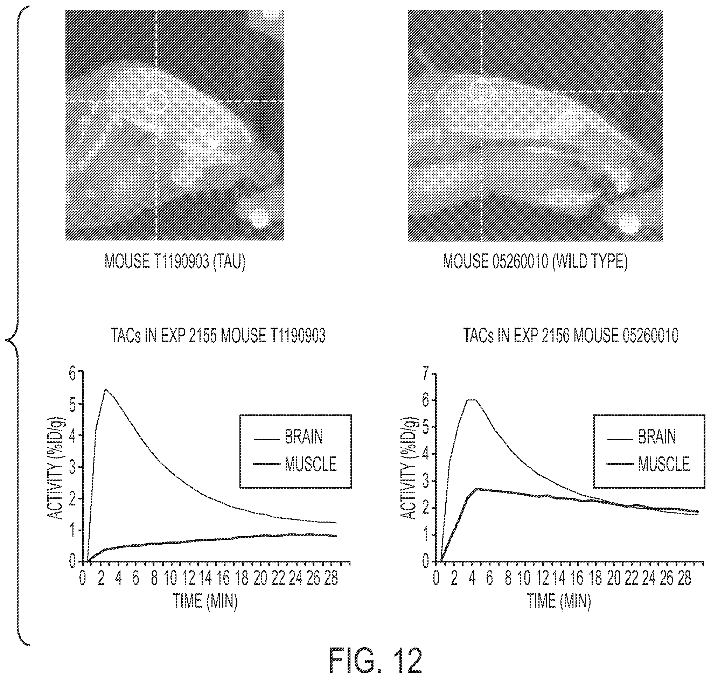

An assay was developed using a Biacore instrument that introduced the ligands over gold-surface immobilized target proteins and measured the resultant rates of association and disassociation in order to test various compounds that bind to soluble AD oligomers, polymers and fibrils (Figures 12 to 17 of USSN 12/372,717).

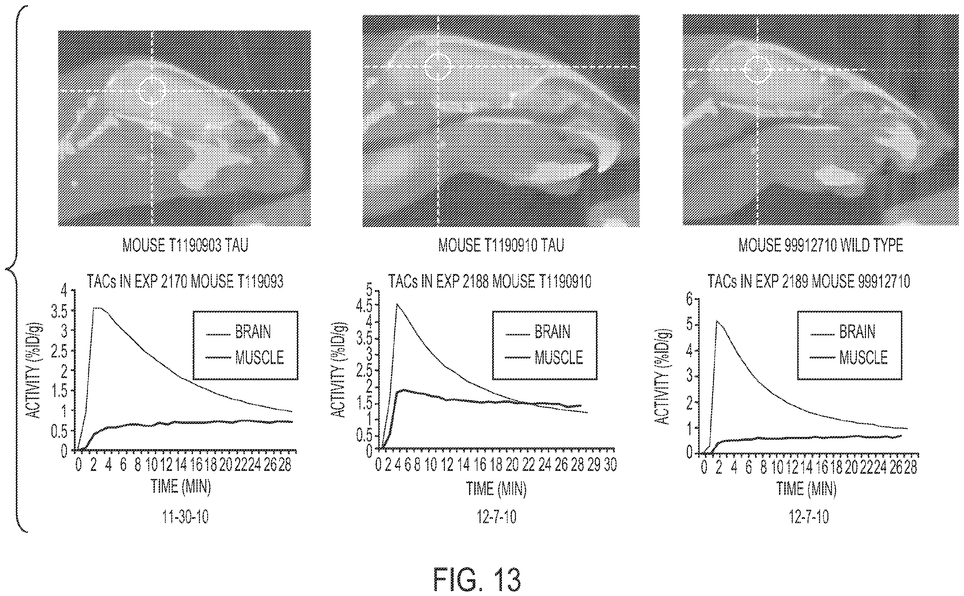

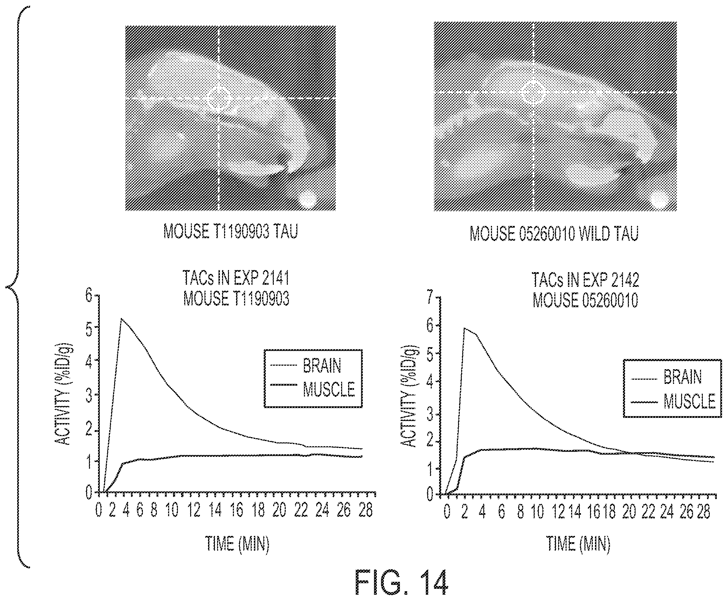

The carbazole series also demonstrated a unique and surprising ability to bind favorably and preferentially to insoluble aggregates (9 nM) over soluble aggregates (262 nM) (Figure 12 and Figure 13 of USSN 12/372,717). PiB also binds well to insoluble aggregates (16 nM) but also binds essentially equally as well to soluble aggregates (48 nM) (Figure 14 and Figure 15 of USSN 12/372,717). For imaging applications where it is favorable to distinguish between a tracer's binding to insoluble versus soluble aggregates, CB-003 provides a larger binding ratio of 29:1, whereas PiB only provides a ratio 3:1. Thus, CB-003 may provide more selective binding information relative to PiB. The results indicate that 1) for soluble aggregate binding, PIB > CB3 > CB4; and 2) for insoluble aggregate binding, PIB = CB3 > CB4.

### MicroPET imaging with [18F]-CB-001 or [18F]-CB-003 in WT and App Mice

The results demonstrate that 1) WT and App mice show statistically significant differences in tracer retention in the brain (Figure 18A, Figure 18B and Figure 19 of USSN 12/372,717); 2) App mice show up to 25% larger brain / muscle ratios compared to WT mice (Figure 20 and Figure 21 of USSN 12/372,717). The carbazoles display both a surprising high uptake in mice brains (both WT and APP) and sufficiently slow washout such that one can distinguish WT from APP mice (Figure 22 and Figure 23 of USSN 12/372,717). Without being bound by any theory proposed herein, we speculate that the reason behind these results may be that CB-003 possesses a faster washout rate than 18F-PiB, which is consistent with consistent with the staining data: 18F-PiB requires harsher wash conditions in order to give reasonable grey to white matter ratios. The rapid washout of CB-003 is presumably a major factor for its low non-specific binding, yet the washout is slow enough to distinguish WT from APP. This suggests that the carbazoles display a unique combination of excellent washout and retention properties in human AD brains that are not obvious from prior art data. CB-003, being a neutral compound, would also potentially possess greater uptake values versus zwitterionic-based imaging agents such as methylene blue.

### General experimental procedure for boronation of arylbromide to arylboronic pinacol ester

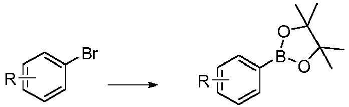

To a microwave vial with a magnetic stir bar, was added the arylbromide starting materials (1 equiv), Pd(dppf)Cl2 (0.05 eq), Potassium acetate (3 eq) and Bis(pinacol)borate (1.2 eq). The solid was dissolved in DMSO (5 vol), sealed and heated to 80°C in an oil bath for 40-50 hours. The reaction was diluted with brine extracted with ether/hexanes or DCM. The combine organic layers was concentrated, the residue was purified over silica gel using Hexanes:EtOAc or DCM:EtOAc or DCM:MeOH as the eluent to afford boronic ester.

### General experimental procedure for cyclization of aza-carbazole from Nitro-substituted biaryl precursor:

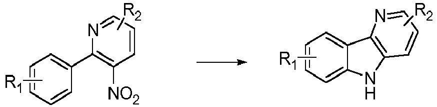

To a microwave vial with a magnetic stir bar, was added the nitro-substituted biaryl precursor aryl/heterocyclic halide (1 equiv), triethyl phosphite (4-8 eq). The suspension was heated at 120-135 °C (depends on the reactivity of the starting material and stability of the product) in an oil bath for 2 hours. The reaction was concentrated under vacuum to remove all the volatiles. The residue was purified over silica gel using Hexanes:EtOAc or DCM:EtOAc or DCM:MeOH as the eluent to afford the aza-carbazoles.

### 7-(6-Fluoropyridine-3-yl)-5-methyl-H-pyrido[4,3-b]indole TFA salt (AS-5357-55, T-820)

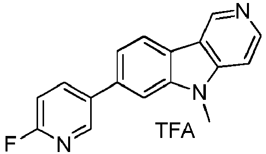

**T-807** 0.010 g was alkylated with dimethylacetone (2 eq) in DMF and Cs₂CO₃ (0.5 eq) at 160°C for 3 hrs. The residue was purified by HPLC using ACN-H₂O with 0.05 %TFA. **T-820** isolated as off white solid 0.006 g (72%); ¹H NMR (400 MHz, CD₃OD): δ 9.58 (s, 1H), 8.64 (d, *J* = 2.4 Hz, 1H), 8.61 (dd, *J* = 6.8 and 0.8 Hz), 8.49 (dd, *J* = 8.4 and 0.8 Hz, 1H), 8.38 (qd, *J* = 8.0 and 2.4 Hz, 1H), 8.15 (d, *J* = 0.8 Hz, 1H), 8.11 (d, *J* = 6.8 Hz, 1H), 7.85 (d, *J* = 8.0 and 1.2 Hz, 1H), 7.23 (dd, *J* = 8.4 and 2.8 Hz, 1H), 4.16 (s, 3H); MS (ESI): 278.1 [+H⁺, Free base].

### 7-(6-Fluoropyridine-3-yl)-5H-pyrido[4,3-b]indole (AS-5357-18, T-807)

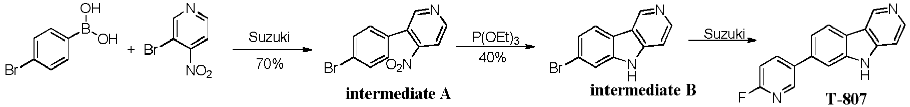

General experimental procedure for Suzuki coupling (Method A) was followed to prepare intermediate **A.** Reaction was performed on a 0.6 g scale. Product eluted out in hexane-EtOAc on a Combiflash purification system, isolated 0.600 g (72%) of intermediate **A** as light yellow solid; MS (ESI): 277 and 279 (M⁺) and (M+2H⁺). Intermediate **A** 0.6 g was cyclized using general method carbazole synthesis (Method CC) afforded carbazole **B.** Carbazole **B** eluted with DCM-MeOH on a Combiflash purification system isolated 0.21 g (40%) as light brown color solid; ¹H NMR (400 MHz, DMSO-d₆): δ 11.9 (s, 1H), 9.36 (d, *J* = 0.88 Hz, 1H), 8.45 (d, *J* = 0.8 Hz, 1H), 8.20 (d, *J* = 8.4 Hz, 1H), 7.76 (d, *J* = 0.8 Hz, 1H), 7.43 (d, *J* = 2.0 Hz, 1H), 7.41 (d, *J* = 1.6 Hz, 1H); MS (ESI): 247 [M⁺] and 249 [M+2H⁺]. Carbazole **B** was further used for Suzuki coupling (Method A). Reaction was performed on a 0.1 g scale. Product **T-807** eluted with DCM-MeOH on a Combiflash purification system, isolated 0.056 g as off white solid (56%) which was further purified by HPLC using ACN-H₂O with 0.05% TFA; ¹H NMR (400 MHz, DMSO-d₆): δ 11.81 (s, 1H), 9.34 (s, 1H), 8.61 (dd, *J* = 1.6 and 0.8 Hz, 1H), 8.41-8.30 (m, 3H), 7.80 (dd, *J* = 4.0 and 0.4 Hz, 1H), 7.57 (dd, *J* = 8.4 and 1.6 Hz, 1H), 7.46 (dd, *J* = 6.4 and 0.8 Hz, 1H), 7.29 (dd, *J* = 8.4 and 2.8 Hz, 1H); MS (ESI): 264.3 [M+H⁺, Free base].

## Claims

1. A compound of the formula: 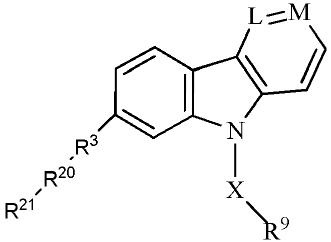 and pharmaceutically acceptable salts and stereoisomers thereof,
wherein:
L is N or CR⁵;
M is N;
R⁵ is H, OH, halo, NH₂, CH₃, NO₂, aryl, C₃-C₈alkenyl, C₃-C₈alkynyl, C₃₋₈cycloalkyl, heteroaryl, N=NR¹², NHR¹², N(R¹²)₂, or (-CH₂)₁₋₁₂-R¹², wherein R¹² is C₁-C₆alkyl, aryl, H or heteroaryl,
wherein at least one H of (-CH₂)₁₋₁₂-R¹², C₃₋₈cycloalkyl, aryl, alkenyl, alkynyl, or heteroaryl, is optionally replaced by halo, OH, NH₂, and C₁₋₈alkyl, wherein at least one H of the C₁₋₈alkyl is optionally replaced by halo, OH or NH₂, and
wherein at least one CH₂ of (-CH₂)₁₋₁₂-R¹² is optionally replaced with C(O), O, S, SO₂, or NH, NH-C₁₋₈alkyl or N(C₁₋₈alkyl)₂, wherein at least one H of the C₁₋₈alkyl is optionally replaced by halo, OH or NH₂,
and wherein at least one CH₂ of the C₃₋₈cycloalkyl is optionally replaced by C(O), O, S, NH or N-C₁₋₈alkyl, wherein at least one H of the C₁₋₈alkyl is optionally replaced by halo or OH,
X is a bond or is C₁₋₁₄alkyl, wherein at least one carbon is optionally replaced by C(O), O, S, SO₂, or NH, NH-C₁₋₈alkyl, and wherein at least one H of C₁₋₈alkyl is optionally replaced by halo, OH, C₁₋₆alkyl;
R⁹ is H, an azide, an alkyne, OH, halo, NH₂, N(C₁₋₈alkyl)₂, aryl or heteroaryl, wherein at least one H of the aryl or heteroaryl is optionally replaced by halo, NH₂, or C₁₋₆ alkyl or C₁₋₆ alkyl, wherein at least one H of the C₁₋₆ alkyl is optionally replaced by halo, or C₃₋₈cycloalkyl, wherein at least one H of the C₃₋₈cycloalkyl is optionally replaced by halo and wherein at least one CH₂ of the C₃₋₈cycloalkyl is optionally replaced with O, OH, S, SH, NH, N-C₁₋₈alkyl;
R³ is a bond or is at least one of O, S, C(O), SO₂, NH, N-C₁₋₈alkyl, (CH₂)₁₋₁₂, wherein at least one C of (CH₂)₁₋₁₂ is optionally replaced by C(O), O, S, SO₂, NH, N-C₁₋₈alkyl and wherein at least one H is optionally replaced by C₁₋₈alkyl or halo,
R²⁰ is aryl or heteroaryl; and
R²¹ is H, OH, halo, NH₂, CH₃, NO₂,, (-CH₂)₁₋₁₂-CH₃, or C₃₋₈cycloalkyl,
wherein at least one H of the (-CH₂)₁₋₁₂-CH₃ or the C₃₋₈cycloalkyl is optionally replaced by halo, OH, NH₂, or C₁₋₈alkyl, wherein at least one H of the C₁₋₈alkyl is optionally replaced by halo or OH,
and wherein at least one CH₂ of the (-CH₂)₁₋₁₂-CH₃ is optionally replaced with C(O), O, S, SO₂, or NH, NH-C₁₋₈alkyl, N(C₁₋₈alkyl)₂, wherein at least one H of the C₁₋₈alkyl is optionally replaced by halo, OH or NH₂,
and wherein at least one CH₂ of the C₃₋₈cycloalkyl is optionally replaced by C(O), O, S, SO₂, or NH, N-C₁₋₈alkyl, wherein at least one H of the C₁₋₈alkyl is optionally replaced by halo, OH or NH₂,
and wherein R²¹ comprises a radionuclide,
provided that the compound is not

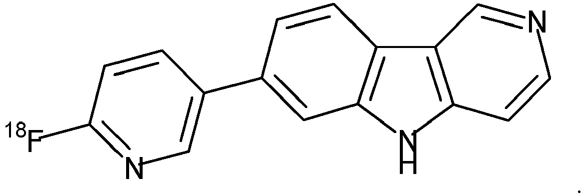

2. The compound of claim 1, wherein R³ is a bond and R²⁰ is heteroaryl.

3. The compound of claim 1, wherein R³ is (CH₂)₁₋₁₂ and wherein at least one C of (CH₂)₁₋₁₂ is optionally replaced by C(O), O, S, SO₂, NH, N-C₁₋₈alkyl and wherein at least one H is optionally replaced by C₁₋₈alkyl or halo.

4. The compound of claim 1 that is: 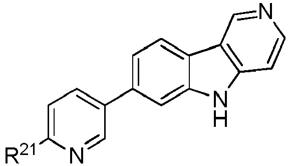 and pharmaceutically acceptable salts and stereoisomers thereof,
wherein R²¹ is a radionuclide.

5. The compound of claim 1 that is:

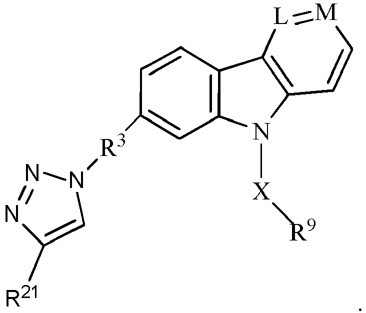

6. The compound of claim 5 wherein R³ is O.

7. The compound of claim 3, wherein R²¹ is C₄-alkyl with at least one CH₂ replaced with NH, at least one CH₂ replaced with C(O) and at least one H replaced by halo.

## Patentansprüche

1. Verbindung der Formel: 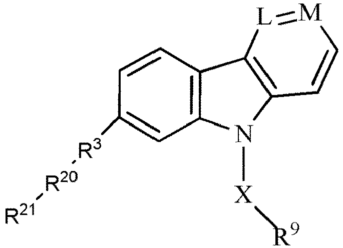 und pharmazeutisch akzeptable Salze und Stereoisomere davon,
wobei:
L N oder CR⁵ ist;
M N ist;
R⁵ H, OH, Halo, NH₂, CH₃, NO₂, Aryl, C₃-C₈-Alkenyl, C₃-C₈-Alkinyl, C₃₋₈-Cycloalkyl, Heteroaryl, N=NR¹², NHR¹², N(R¹²)₂ oder (-CH₂)₁₋₁₂-R¹² ist, wobei R¹² C₁-C₆-Alkyl, Aryl, H oder Heteroaryl ist,
wobei mindestens ein H von (-CH₂)₁₋₁₂-R¹², C₃₋₈-Cycloalkyl, Aryl, Alkenyl, Alkinyl oder Heteroaryl optional durch Halo, OH, NH₂ und C₁₋₈-Alkyl ersetzt ist, wobei mindestens ein H des C₁₋₈-Alkyls optional durch Halo, OH oder NH₂ ersetzt ist, und
wobei mindestens ein CH₂ von (-CH₂)₁₋₁₂-R¹² optional durch C(O), O, S, SO₂ oder NH, NH-C₁₋₈-Alkyl oder N(C₁₋₈-Alkyl)₂ ersetzt ist, wobei mindestens ein H des C₁₋₈-Alkyls optional durch Halo, OH oder NH₂ ersetzt ist,
und wobei mindestens ein CH₂ des C₃₋₈-Cycloalkyls optional durch C(O), O, S, NH oder N-C₁₋₈-Alkyl ersetzt ist, wobei mindestens ein H des C₁₋₈-Alkyls optional durch Halo oder OH ersetzt ist,
X eine Bindung ist oder C₁₋₁₄-Alkyl ist, wobei mindestens ein Kohlenstoff optional durch C(O), O, S, SO₂ oder NH, NH-C₁₋₈-Alkyl ersetzt ist und wobei mindestens ein H des C₁₋₈-Alkyls optional durch Halo, OH, C₁₋₆-Alkyl ersetzt ist;
R⁹ H, ein Azid, ein Alkin, OH, Halo, NH₂, N(C₁₋₈-Alkyl)₂, Aryl oder Heteroaryl ist,
wobei mindestens ein H des Aryls oder Heteroaryls optional durch Halo, NH₂ oder C₁₋₆-Alkyl oder C₁₋₆-Alkyl ersetzt ist, wobei mindestens ein H des C₁₋₆-Alkyls optional durch Halo oder C₃₋₈-Cycloalkyl ersetzt ist, wobei mindestens ein H des C₃₋₈-Cycloalkyls optional durch Halo ersetzt ist und wobei mindestens ein CH₂ des C₃₋₈-Cycloalkyls optional durch O, OH, S, SH, NH, N-C₁₋₈-Alkyl ersetzt ist;
R³ eine Bindung ist oder mindestens eines von O, S, C(O), SO₂, NH, N-C₁₋₈-Alkyl, (CH₂)₁₋₁₂ ist, wobei mindestens ein C von (CH₂)₁₋₁₂ optional durch C(O), O, S, SO₂, NH, N-C₁₋₈-Alkyl ersetzt ist und wobei mindestens ein H optional durch C₁₋₈-Alkyl oder Halo ersetzt ist,
R²⁰ Aryl oder Heteroaryl ist und
R²¹ H, OH, Halo, NH₂, CH₃, NO₂, (-CH₂)₁₋₁₂-CH₃ oder C₃₋₈-Cycloalkyl ist,
wobei mindestens ein H des (-CH₂)₁₋₁₂-CH₃ oder des C₃₋₈-Cycloalkyls optional durch Halo, OH, NH₂ oder C₁₋₈-Alkyl ersetzt ist, wobei mindestens ein H des C₁₋₈-Alkyls optional durch Halo oder OH ersetzt ist,
und wobei mindestens ein CH₂ des (-CH₂)₁₋₁₂-CH₃ optional durch C(O), O, S, SO₂ oder NH, NH-C₁₋₈-Alkyl, N(C₁₋₈-Alkyl)₂ ersetzt ist, wobei mindestens ein H des C₁₋₈-Alkyls optional durch Halo, OH oder NH₂ ersetzt ist,
und wobei mindestens ein CH₂ des C₃₋₈-Cycloalkyls optional durch C(O), O, S, SO₂ oder NH, N-C₁₋₈-Alkyl ersetzt ist, wobei mindestens ein H des C₁₋₈-Alkyls optional durch Halo, OH oder NH₂ ersetzt ist,
und wobei R²¹ ein Radionuklid umfasst,
vorausgesetzt, dass die Verbindung nicht 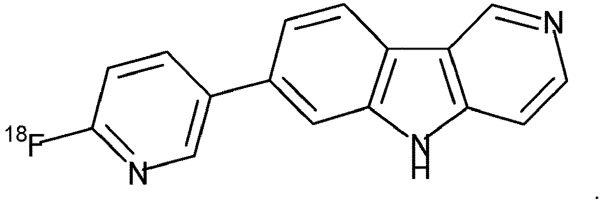
ist.

2. Verbindung nach Anspruch 1, wobei R³ eine Bindung ist und R²⁰ Heteroaryl ist.

3. Verbindung nach Anspruch 1, wobei R³ (CH₂)₁₋₁₂ ist und wobei mindestens ein C von (CH₂)₁₋₁₂ optional durch C(O), O, S, SO₂, NH, N-C₁₋₈-Alkyl ersetzt ist und wobei mindestens ein H optional durch C₁₋₈-Alkyl oder Halo ersetzt ist.

4. Verbindung nach Anspruch 1, die: 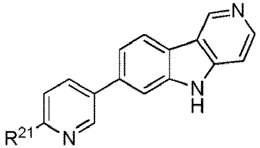 ist, und pharmazeutisch akzeptable Salze und Stereoisomere davon, wobei R²¹ ein Radionuklid ist.

5. Verbindung nach Anspruch 1, die: 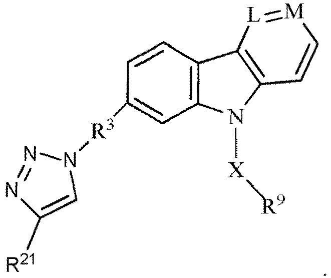 ist.

6. Verbindung nach Anspruch 5, wobei R³ O ist.

7. Verbindung nach Anspruch 3, wobei R²¹ C₄-Alkyl ist, wobei mindestens ein CH₂ durch NH ersetzt ist, mindestens ein CH₂ durch C(O) ersetzt ist und mindestens ein H durch Halo ersetzt ist.

## Revendications

1. Composé répondant à la formule : 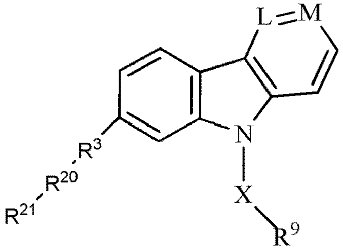 et des sels pharmaceutiquement acceptables et des stéréoisomères de celui-ci,
dans lequel :
L représente N ou CR⁵ ;
M représente N ;
R⁵ représente H, OH, halo, NH₂, CH₃, NO₂, un aryle, un alcényle en C₃ à C₈, un alcynyle en C₃ à C₈, un cycloalkyle en C₃ à C₈, un hétéroaryle, N=NR¹², NHR¹², N(R¹²)₂ ou (-CH₂)₁₋₁₂-R¹², dans lequel R¹² représente un alkyle en C₁ à C₆, un aryle, H ou un hétéroaryle,
dans lequel au moins un H du (-CH₂)₁₋₁₂-R¹², cycloalkyle en C₃ à C₈, aryle, alcényle, alcynyle ou hétéroaryle est éventuellement remplacé par halo, OH, NH₂ et un alkyle en C₁ à C₈, dans lequel au moins un H de l'alkyle en C₁ à C₈ est éventuellement remplacé par halo, OH ou NH₂, et
dans lequel au moins un CH₂ du (-CH₂)₁₋₁₂-R¹² est éventuellement remplacé par C(O), O, S, SO₂ ou NH, NH-alkyle en C₁ à C₈ ou N(alkyle en C₁ à C₈)₂, dans lequel au moins un H de l'alkyle en C₁ à C₈ est éventuellement remplacé par halo, OH ou NH₂,
et dans lequel au moins un CH₂ du cycloalkyle en C₃ à C₈ est éventuellement remplacé par C(O), O, S, NH ou N-alkyle en C₁ à C₈, dans lequel au moins un H de l'alkyle en C₁ à C₈ est éventuellement remplacé par halo ou OH,
X représente une liaison ou représente un alkyle en C₁ à C₁₄, dans lequel au moins un carbone est éventuellement remplacé par C(O), O, S, SO₂ ou NH, NH-alkyle en C₁ à C₈, et dans lequel au moins un H de l'alkyle en C₁ à C₈ est éventuellement remplacé par halo, OH, un alkyle en C₁ à C₆ ;
R⁹ représente H, un azide, un alcyne, OH, halo, NH₂, N(alkyle en C₁ à C₈)₂, un aryle ou un hétéroaryle, dans lequel au moins un H de l'aryle ou de l'hétéroaryle est éventuellement remplacé par halo, NH₂ ou un alkyle en C₁ à C₆, dans lequel au moins un H de l'alkyle en C₁ à C₆ est éventuellement remplacé par halo ou un cycloalkyle en C₃ à C₈, dans lequel au moins un H du cycloalkyle en C₃ à C₈ est éventuellement remplacé par halo et dans lequel au moins un CH₂ du cycloalkyle en C₃ à C₈ est éventuellement remplacé par O, OH, S, SH, NH, N-alkyle en C₁ à C₈ ;
R³ représente une liaison ou représente au moins l'un parmi O, S, C(O), SO₂, NH, N-alkyle en C₁ à C₈, (CH₂)₁₋₁₂, dans lequel au moins un C du (CH₂)₁₋₁₂ est éventuellement remplacé par C(O), O, S, SO₂, NH, N-alkyle en C₁ à C₈ et dans lequel au moins un H est éventuellement remplacé par un alkyle en C₁ à C₈ ou halo,
R²⁰ représente un aryle ou un hétéroaryle ; et
R²¹ représente H, OH, halo, NH₂, CH₃, NO₂, (-CH₂)₁₋₁₂-CH₃ ou un cycloalkyle en C₃ à C₈,
dans lequel au moins un H du (-CH₂)₁₋₁₂-CH₃ ou du cycloalkyle en C₃ à C₈ est éventuellement remplacé par halo, OH, NH₂ ou un alkyle en C₁ à C₈, dans lequel au moins un H de l'alkyle en C₁ à C₈ est éventuellement remplacé par halo ou OH,
et dans lequel au moins un CH₂ du (-CH₂)₁₋₁₂-CH₃ est éventuellement remplacé par C(O), O, S, SO₂ ou NH, NH-alkyle en C₁ à C₈, N(alkyle en C₁ à C₈)₂, dans lequel au moins un H de l'alkyle en C₁ à C₈ est éventuellement remplacé par halo, OH ou NH₂,
et dans lequel au moins un CH₂ du cycloalkyle en C₃ à C₈ est éventuellement remplacé par C(O), O, S, SO₂ ou NH, N-alkyle en C₁ à C₈, dans lequel au moins un H de l'alkyle en C₁ à C₈ est éventuellement remplacé par halo, OH ou NH₂,
et dans lequel R²¹ comprend un radionucléide, à condition que le composé ne soit pas

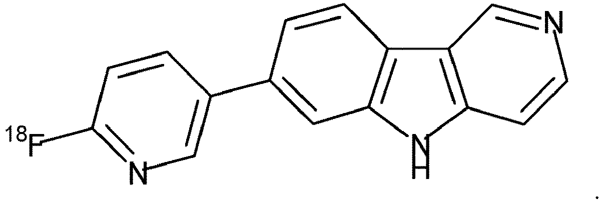

2. Composé selon la revendication 1, dans lequel R³ représente une liaison et R²⁰ représente un hétéroaryle.

3. Composé selon la revendication 1, dans lequel R³ représente (CH₂)₁₋₁₂ et dans lequel au moins un C du (CH₂)₁₋₁₂ est éventuellement remplacé par C(O), O, S, SO₂, NH, N-alkyle en C₁ à C₈ et dans lequel au moins un H est éventuellement remplacé par un alkyle en C₁ à C₈ ou halo.

4. Composé selon la revendication 1 qui est : 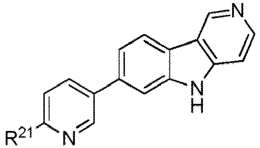 et des sels pharmaceutiquement acceptables et des stéréoisomères de celui-ci,
dans lequel R²¹ représente un radionucléide.

5. Composé selon la revendication 1 qui est :

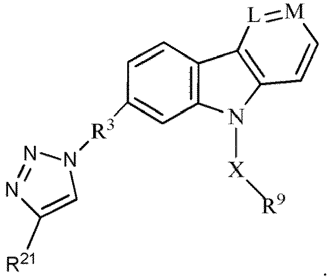

6. Composé selon la revendication 5, dans lequel R³ représente O.

7. Composé selon la revendication 3, dans lequel R²¹ représente un alkyle en C₄ avec au moins un CH₂ remplacé par NH, au moins un CH₂ remplacé par C(O) et au moins un H remplacé par halo.
